# EUROPEAN PATENT SPECIFICATION

(11) **EP 1 579 004 B1**
(45) Date of publication and mention of the grant of the patent: **16.06.2010**
(21) Application number: 03779324.7
(22) Date of filing: 23.10.2003
(51) Int. Cl.: C12Q 1/68

(54) **SCREENING METHODS FOR IDENTIFICATION OF EFFICIENT PRE-TRANS-SPLICING MOLECULES**
SCREENING-VERFAHREN ZUR IDENTIFIZIERUNG WIRKSAMER PRÄ-TRANS-SPLEISSMOLEKÜLE
PROCEDES DE CRIBLAGE DESTINES A IDENTIFIER DES MOLECULES DE PRE-TRANS-EPISSAGE EFFICACES

(30) Priority: 23.10.2002 US 420498 P
(43) Date of publication of application: 28.09.2005
(73) Proprietor: VIRxSYS Corporation, Gaithersburg, MD 20877 (US)
(72) Inventor: MITCHELL, Lloyd, G., Bethesda, MD 20814 (US); PUTTARAJU, Madaiah, Germantown, MD 20876 (US); GARCIA-BLANCO, Mariano, Durham, NC 27713 (US); OTTO, Edward, Great Falls, VA 22066 (US); YANG, Yanping, Rockville, MD 20850 (US)
(74) Representative: Irvine, Jonquil Claire
(86) International application number: PCT/US2003/034102
(87) International publication number: WO 2004/038380

(56) References cited:
- US-A- 6 013 487
- US-A1- 2002 015 979
- US-A1- 2002 115 207
- US-A1- 2002 115 207
- MARTINEZ-SALAS ENCARNACION: "INTERNAL RIBOSOME ENTRY SITE BIOLOGY AND ITS USE IN EXPRESSION VECTORS" CURRENT OPINION IN BIOTECHNOLOGY, LONDON, GB, vol. 10, no. 5, 1999, pages 458-464, XP000943467 ISSN: 0958-1669
- KIKUMORI T ET AL: "Promiscuity of pre-mRNA spliceosome-mediated trans splicing: A problem for gene therapy?" HUMAN GENE THERAPY, MARY ANN LIEBERT, NEW YORK ,NY, US, vol. 12, 20 July 2001 (2001-07-20), pages 1429-1441, XP002974762 ISSN: 1043-0342
- DAVIS, ET AL.: 'RNA Trans-splicing in Flatworms' THE JOURNAL OF BIOLOGICAL CHEMISTRY vol. 270, no. 37, September 1995, pages 21813 - 21819, XP002985053

## Description

### 1. INTRODUCTION

The present invention provides methods and compositions for rapid high capacity functional screening for optimal pre-trans-splicing molecules (PTMs). The compositions of the invention include PTM expression libraries capable of encoring candidate PTMs designed to interact with a target precursor messenger RNA molecule (target pre-mRNA) and mediate a trans-splicing reaction resulting in the generation of a novel chimeric RNA molecule (chimeric RNA).

### 2. BACKGROUND OF THE INVENTION

DNA sequences in the chromosome are transcribed into pre-mRNAs which contain coding region (exons) and generally also contain intervening non-coding regions (introns). Introns are removed from pre-mRNAs in a precise process called splicing (Chow et al., 1977, Cell 12:1-8; and Berger, S.M. et al., 1977, Proc. NatL Acad. Sci. USA 74:3171-3175). Splicing takes place by the coordinated interaction of several small nuclear ribonucleoprotein particles (snRNP's) and many protein factors that assemble to form an enzymatic complex known as the spliceosome (Moore et al., 1993, in The RNA World, R.F. Gestland and J.F. Atkins eds. (Cold Spring Harbor Laboratory Press, Cold Spring Harbor, N.Y.); Kramer, 1996, Ann. Rev. Biochem., 65:367-404; Staley and Guthrie, 1998, Cell 92:315-326).

In most cases, the splicing reaction occurs within the same pre-mRNA molecule, which is termed cis-splicing. Splicing between two independently transcribed pre-mRNAs is termed *trans*-splicing. *Trans*-splicing was first discovered in trypanosomes (Sutton & Boothroyd, 1986, Cell 47:527; Murphy et al., 1986, Cell 47:517) and subsequently in nematodes (Krause & Hirsh, 1987, Cell 49:753); flatworms (Rajkovic et al., 1990, Proc. Natl. Acad. Sci. USA, 87:8879; Davis et al., 1995, J. Biol. Chem. 270:21813) and in plant mitochondria (Malek et al.. 1997, Proc. Natl. Acad. Sci. USA 94:553). This form of *trans*-splicing requires specialized pre-mRNAs.

*Trans*-splicing may also refer to a different process which occurs between two conventional pre-mRNAs, where an intron of one pre-mRNA interacts with an intron of a second pre-mRNA, enhancing the recombination of splice sites between two conventional pre-mRNAs. This type of *trans*-splicing was postulated to account for transcripts encoding a human immunoglobulin variable region sequence linked to the endogenous constant region in a transgenic mouse (Shimizu et al., 1989, Proc. Nat'l Acad. Sci. USA 86:8020). In addition, *trans*-splicing of c-myb pre-RNA has been demonstrated (Vellard, M. et al. Proc. Natl. Acad. Sci., 1992 89:2511-2515) and more recently, RNA transcripts from cloned SV40 *trans*-spliced to each other were detected in cultured cells and nuclear extracts (Eul et al., 1995, EMBO. J. 14:3226). Recent reports of endogenous *trans*-splicing include those of Takahara T et al., (2002 Biochem Biophys Res Commun. 298:156); Flouriot G, et al. (2002 J Biol Chem. 277:26244-51); and Kikumori T, et al. (2002 FEBS Lett. 522:41-6). However, naturally occurring *trans*-splicing of mammalian pre-mRNAs is thought to be an exceedingly rare event.

*In vitro trans*-splicing in cell free nuclear extracts has been used as a model system to examine the mechanism of splicing by several groups (Konarska & Sharp, 1985, Cell 46:165-171 Solnick, 1985, Cell 42:157; Chiara & Reed, 1995, Nature 375:510) Reasonably efficient *trans*-splicing (30% of cis-spliced analog) was achieved between RNAs capable of base pairing to each other, while splicing of RNAs not tethered by base pairing was diminished by a factor of 10. Other *in vitro trans*-splicing reactions not requiring obvious RNA-RNA interactions among the substrates were observed by Chiara & Reed (1995, Nature 375:510), Bruzik J.P. and Maniatis T. (1992, Nature 360: 692) and Bruzik J.P. and Maniatis T. (1995 Proc. Natl. Acad. Sci USA 92:7056-7059). These reactions occur at relatively low frequencies and require specialized elements, such as a downstream 5' splice site or exonic splicing enhancers.

Until recently, the practical application of targeted *trans*-splicing to modify specific target genes has been limited to group I ribozyme-based mechanisms. Using the *Tetrahymena* group I ribozyme, targeted *trans*-splicing was demonstrated in *E. coli.* (Sullenger B.A. and Cech. T.R., 1994, Nature 341:619-622), in mouse fibroblasts (Jones, J.T. et al.,1996, Nature Medicine 2:643-648), human fibroblasts (Phylacton, L.A. et al. Nature Genetics 18:378-381) and human erythroid precursors (Lan et al., 1998, Science 280:1593-1596).

Spliceosome mediated RNA *trans*-splicing is a novel platform technology with broad applications that include RNA therapy, suicide gene therapy, molecular evolution and real time imaging of gene expression in live cells (Otto et al., Current Drug Discovery 2003). Spliceosome mediated RNA *Trans*-splicing is accomplished by the introduction of RNAs called pre-trans-splicing molecules (PTMs) into a cell. U.S. Patent Nos. 6,083,702, 6,013,487 and 6,280,978 describe the use of PTMs to mediate *trans*-splicing reactions by contacting a target precursor mRNA to generate novel chimeric RNAs.

PTMs typically have 3 modular domains that consist of an anti-sense binding domain (BD), a splice site and an encoded gene to be trans-spliced. Targeting different genes is accomplished by changing the binding domain. Changing the *trans-*spliced sequence delivered by the PTM allows its use for different applications. The splice site of the PTM recruits the endogenous splicing machinery of the cell, the spliceosome, to carry out this process. Human cells contain the components to form an average of 100,000-200,000 spliceosomes per cell. To date, spliceosome mediated RNA *trans*-splicing has been used for suicide gene therapy by *trans*-splicing the coding sequence of potent toxins into a cancer associated pre-mRNA, BHCG6, *in vitro* and *in vivo* (Puttaraju et al., Nature Biotechnology 1999). In addition, spliceosome mediated RNA *trans*-splicing therapy has corrected mutations that cause cystic fibrosis (CF), restoring therapeutic levels of chloride channel activity in human polarized CF airway epithelia cell cultures and human CF bronchial xenografts (Liu X. et al., Nature Biotechnology 2002 20:47-52) and to restore factor VIII production in hemophilia A knockout mice and correct the phenotype (Chao H. et al., Nature Medicine 2003 9:1015-1019). In culture model systems targeting human papilloma virus (HPV) sequences, *trans*-splicing efficiencies up to 80% has been achieved in cells co-transfected with target and PTM plasmids and 15% in cells containing PTMs and endogenously produced HPV pre-mRNA (unpublished data). In addition, *trans-*splicing has also been demonstrated in a *trans*genic mouse model that produced the 5'portion of the lacZ gene upstream of a portion of the human CFTR intron 9. PTMs targeting CFTR intron 9 and encoding the 3' portion of LacZ sequence were delivered to the lung epithelium by adenovirus or AAV, repairing the endogenous pre-mRNA target and restoring β-galactosidase function. Finally, PTMs encoding reporter genes have been successfully used to image expression of a hemi-reporter gene in mice (Bhaumik et al. Mol. Imaging and Biology 2002; Bhaumik et al. Mol. Therapy 2003). US Patent Application Publication 2002/0115207 (see also WO 02/053581) describes PTM expression libraries, stating that they can encode a translatable protein that acts as a marker, including marker proteins or peptides which may be used to identify or image cells. These results demonstrate the usefulness of spliceosome mediated RNA *trans-*splicing technology for various applications.

Although, *trans*-splicing efficiencies up to 80% in model systems and ~15% when targeting endogenous mRNAs have been achieved to date, further improvements in *trans*-splicing efficiency and specificity may be desired. In targeting HPV, sequence analysis of randomly selected tram-spficed clones repealed that the specificity of *trans*-splicing was ~53% in model systems where the target and PTM were co-delivered. Specificity dropped to <1% when an endogenous HPV mRNA was targeted. Similar results were obtained with PTMs targeted to endogenous ErbB2 pre-mRNA. An independent study by Kikumori *et al*., (2001) also reported low levels of *trans*-splicing specificity. These findings strongly suggest that a high throughput screen that permits rapid testing of very large sequence combinations may be useful to identify PTM molecules with both high specificity and efficiency.

Currently, there are no rapid high-capacity functional screening methods available to screen or evaluate PTMs on the basis of *trans*-splicing efficiency and specificity. The main advantages conferred by a high capacity screen are (i) the testing of 10⁶ or more sequence combinations in a single screen compared to testing only a few individual rationally constructed PTMs in the same time frame, (ii) no requirement for prior knowledge of important sequence elements that influence *trans-*splicing, and (iii) a variety of sequence elements, (e.g., BD length, secondary structure, strength of the 3' splice site, spacer length, etc) can be evaluated simultaneously. The present invention provides novel efficient methods and compositions for evaluating and identifying PTMs with increased specificity and efficiency. For genes with small or large pre-mRNAs, especially those with large introns, the present invention can be used to identify which intron and more specifically, where in that intron is the best region to target binding of the PTM to efficiently produce a *trans*-splicing reaction.

### SUMMARY OF THE INVENTION

The present invention provides a method for identifying optimal pre-trans-splicing molecules (PTMs) capable of interacting with a target pre-mRNA and of mediating a trans-splicing reaction therewith, resulting in the generation of a chimeric RNA molecule, the method comprising:
(I) contacting an expression library of candidate PTMs with cells expressing a target pre-mRNA under conditions in which such a trans-splicing reaction will occur in the presence of one or more PTMs, wherein said candidate PTMs comprise (i) a target-binding domain; (ii) a 3'-splice region and/or a 5'-splice region; (iii) at least one nucleic acid sequence encoding a portion of a first reporter molecule, so that a successful trans-splicing reaction will result in the formation of a chimeric RNA molecule encoding a complete first reporter molecule, (iv) a nucleic acid sequence encoding a different, second reporter molecule and (v) an internal ribosome entry site (IRES), whereby the expression of the nucleic acid sequence encoding the second reporter molecule will be driven off the IRES; but wherein at least one structural element of the candidate PTMs, selected from (a) target-binding domain, (b) 3' splice region and/or 5' splice region, (c) optional spacer region that separates the splice region from the target-binding domain and (d) optional safety sequence, is replaced library-wide by random or a multiplicity of nucleotide sequences;
(II) selecting cells expressing the first and second reporter molecules, thus indicating the presence of a PTM capable of mediating a trans-splicing reaction in the selected cell and the specificity of the trans-splicing; and
(III) identifying the PTM expressed in the selected cells.

The invention also includes the expression library defined in (I) above and a culture of mammalian cells which contains members of such an expression library.

Structural elements normally associated with PTMs include target binding domains, 3' splice regions, 5' splice regions, spacer regions that separate the RNA splice site from the target binding and "safety sequences", to name a few. The candidate PTMs of the invention contain one or more of these structural elements replaced with random nucleotide sequences or nucleotide sequences derived from a chosen gene sequence. The use of such nucleotide sequences is designed to generate a vast array of candidate PTMs with different *trans*-splicing capabilities. Additionally, the candidate PTMs of the invention are designed to encode reporter molecules that can be used to select for cells expressing optimal PTMs. In some instances, the reporter molecule itself may provide the detectable signal, while in other cases a reporter probe, having an affinity for the reporter molecule will provide the detectable signal. The candidate PTMs further comprise internal ribosome entry sites designed to promote expression of a second (or additional), full-length reporter molecule for evaluation of *trans*-splicing specificity. Splicing promotes export of mRNA to the cytoplasm. Expression of the repaired PTM reporter gene should therefore be correlated with the level (efficiency) of *trans*-splicing and, expression of the full-length reporter in the absence of target, should be useful in assessing the specificity of PTM *trans*-splicing and direct PTM expression.

The compositions and screening methods of the invention can be used to rapidly evaluate, compare and identify optimal PTMs on the basis of their ability to mediate an efficient and specific *trans*-splicing reaction. In particular, the present invention provides a means for functionally evaluating large libraries, *i.e*., 10⁶-10⁷ or more, of candidate PTMs generated by modifying various elements or regions of the PTM.

The methods and compositions of the invention can be used to identify optimal PTMs for use in gene regulation, gene repair and targeted cell death. Such methods and compositions can be used for the treatment of various disease including, but not limited to, genetic infectious or autoimmune diseases and proliferative disorder such as cancer, e.g. by suicide gene therapy, and to regulate gene expression in plants.

### 4. BRIEF DESCRIPTION OF THE DRAWINGS

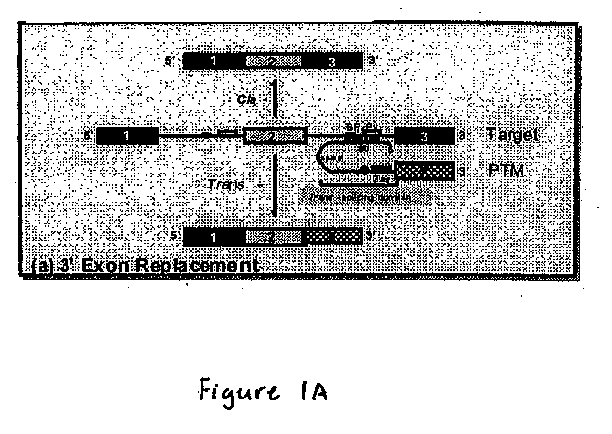

Figure 1A. Model of 3' exon replacement spliceosome-mediated RNA *trans*-splicing. Efficient trans-splicing repairs the hemi-reporter.

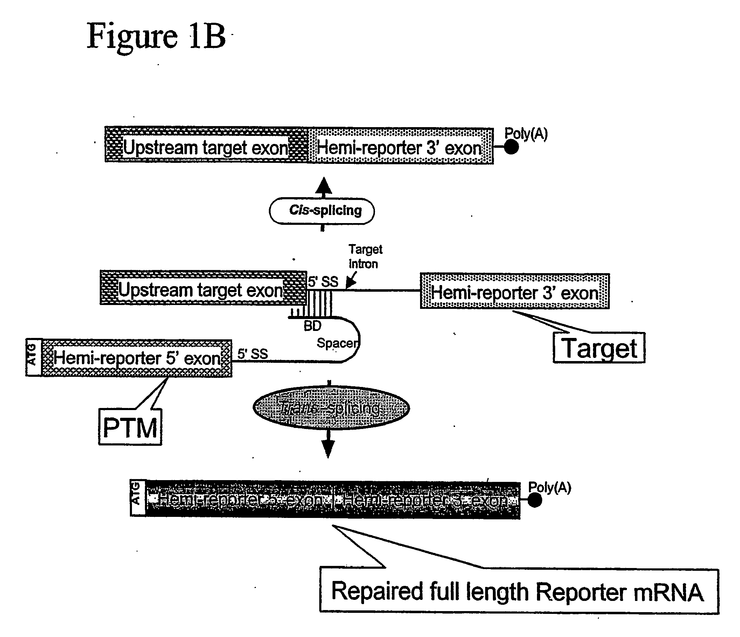

Figure 1B. Model of 5' exon replacement spliceosome-mediated RNA *trans*-splicing. Efficient trans-splicing repairs the hemi-reporter.

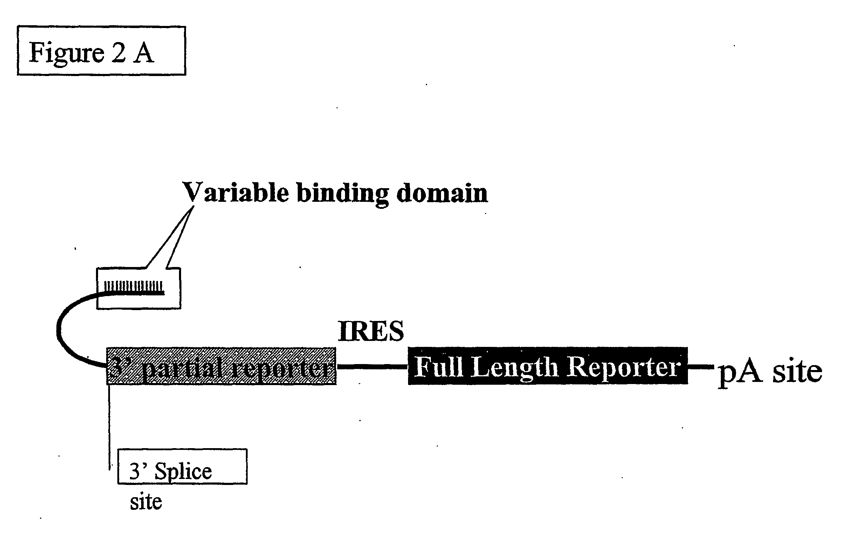

Figure 2A Model of prototype 3' PTM cassette for inserting in binding domain libraries. 3' PTM cassette containing (i) variable binding domain sequence derived from random sequences derived from the target gene (approximately 20-300 base pairs); (ii) sequences encoding a portion of a reporter molecule (hemi-reporter) to assess efficiency of *trans*-splicing (iii) an internal ribosome entry site (IRES); and (iv) sequences encoding a full-length reporter to assess the specificity of *trans*-splicing.

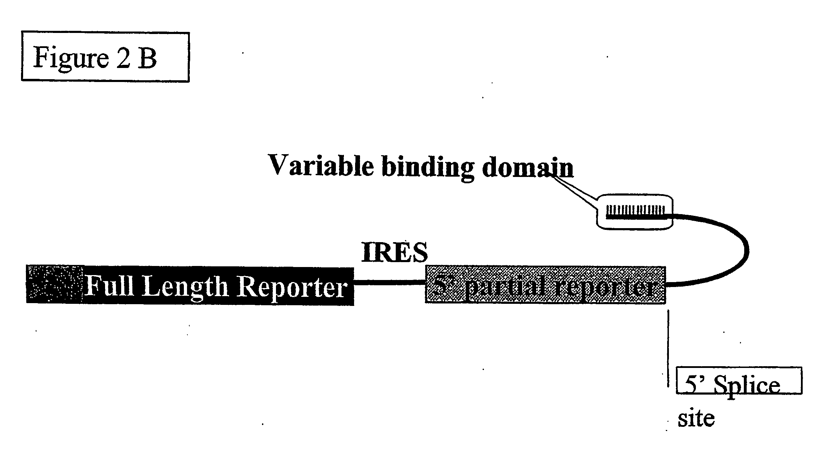

Figure 2B. Model of prototype 5' PTM cassette for inserting in binding domain libraries. 5' PTM cassette containing (i) variable binding domain sequences derived from random sequences derived from the target gene (approximately 20-300 base pairs); (ii) sequences encoding a portion of a reporter molecule (hemi reporter) to assess efficiency of *trans*-splicing; (iii) an internal ribosome entry site (IRES); and (iv) sequences encoding a full-length reporter to assess the specificity of splicing.

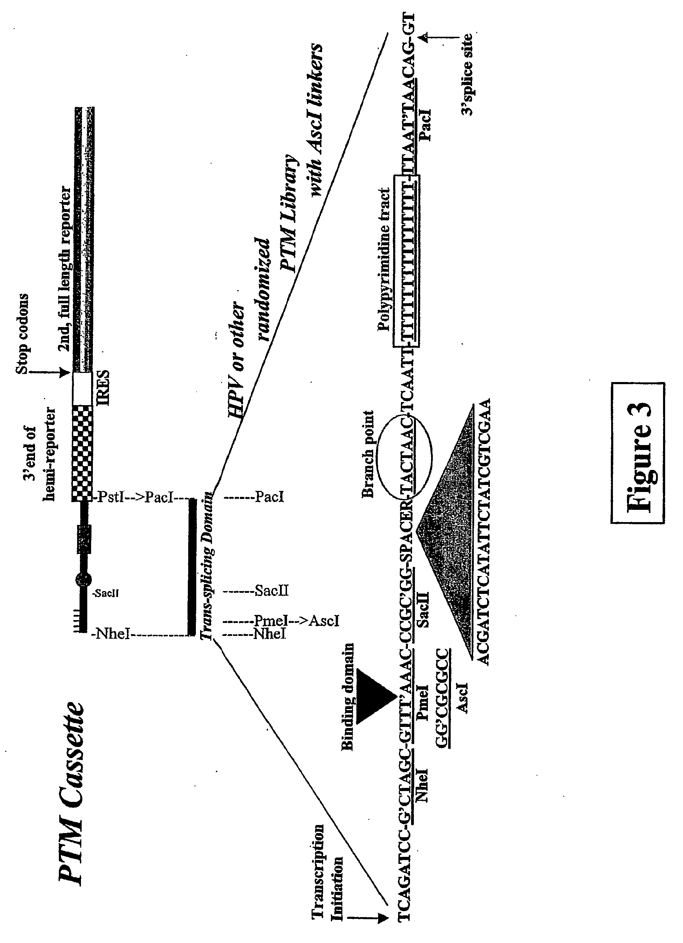

Figure 3. PTM cassette for construction of libraries containing variable binding domains. PTM comprises sequences including restriction enzyme sites to facilitate cloning of variable binding domains, spacer region, 3' splice elements, including for example, splicing branch point, polypyrimidine tract and acceptor AG dinucleotide.

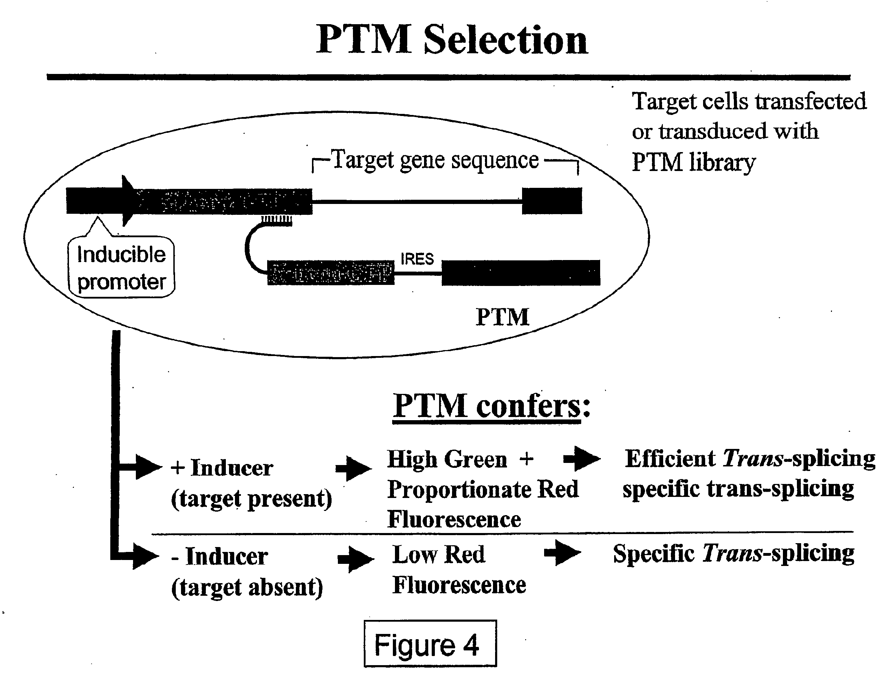

Figure 4. Schematic representation of PTM selection. Target cells are transfected or transduced with a library of PTMs comprising sequences encoding a portion of a first reporter molecule (e.g., a hemi- or partial green florescent protein, GFP), an IRES, and a second full-length reporter molecule (e.g., red florescent protein, RFP). The target cells express a target pre-mRNA that is under the control of an inducible promoter comprising sequences encoding the remaining portion of the first reporter (e.g., the region of the hemi-green florescent protein not encoded by the PTM), a target intron and a terminal exon(s). In the presence of inducer, the target pre-mRNA is expressed. *Trans*-splicing of the candidate PTMs to the target pre-mRNA results in expression of the first reporter molecule (GFP), the level of which is indicative of the efficiency of specific *trans*-splicing. In the presence of target hemi-reporter pre-mRNA, expression of the repaired hemi-reporter (GFP) will be produced by specific *trans*-splicing; expression of the PTM encoded full length reporter(s) (second reporter molecule, in this example RFP) driven off the IRES sequence will be the result of specific *trans*-splicing, as well as non-specific *trans*-splicing and direct PTM expression. An efficient PTM will produce high levels of the repaired hemi-reporter and low (proportionate) levels of the second full length reporter. A completely specific PTM should express a level of the second reporter that would be equivalent to the level of expression of the repaired hemi-reporter (taking into account any effect that the IRES has upon expression of the full length reporter relative to the expression of the repaired hemi-reporter). In the absence of inducer, the target pre-mRNA is not expressed, and the level of expression of the second reporter (RFP) is correlated with the specificity of *trans*-splicing. In the absence of target pre-mRNA, the level of expression of the full (second) length PTM encoded reporter will be the result of non-specific *trans*-splicing or direct PTM expression. This method can facilitate the identification of PTMs with desired or optimal specificity. Testing in the presence and absence of target induction allows the assessment of the specificity of *trans*-splicing.

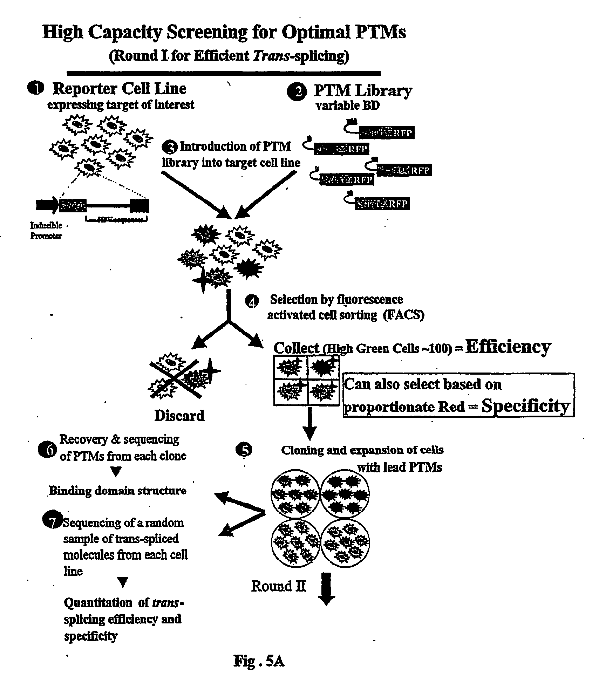

Figure 5A is a representation of round I of the high capacity screening method designed for identification of optimal PTMs. Round I of the screen is designed to identify PTMs that *trans*-splice with high efficiency, as indicated by high levels of expression of a first reporter molecule (e.g. GFP). The relative specificity of the PTMs can also be assessed, as expression of RFP to levels disproportionate (in excess to the level of GFP produced by the PTM) to the level of GFP produced is indicative of an additional contribution from either non-specific trans-splicing or direct PTM expression.

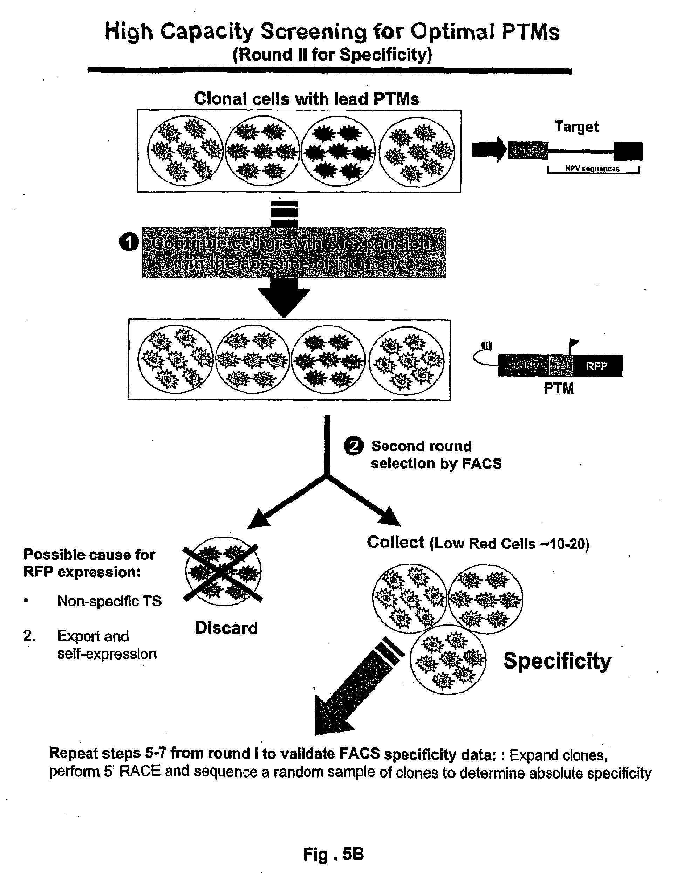

Figure 5B is a representation of round II of the high capacity screening method designed for identification of optimal PTMs. Round II of the screen is designed to identify PTMs that *trans*-splice with high specificity, as indicated by low levels of expression of a second reporter (full length reporter(s) encoded by the PTM, e.g. RFP). The sequential combination of round I followed by round II yields (can be used to identify) PTMs with both high efficiency and specificity.

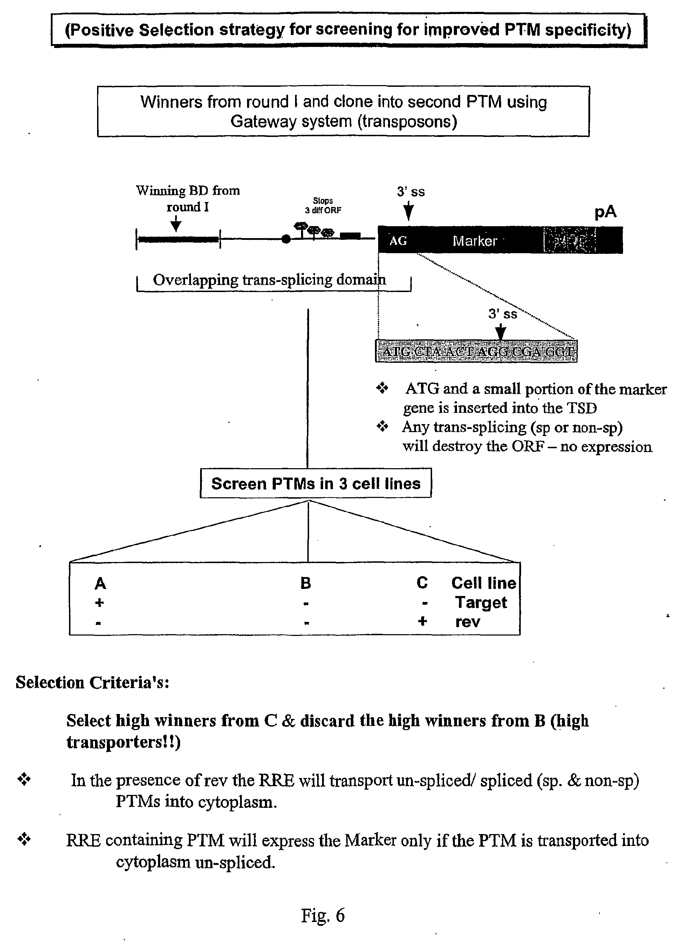

Figure 6 is a representation of an alternative positive selection strategy for round II screening.

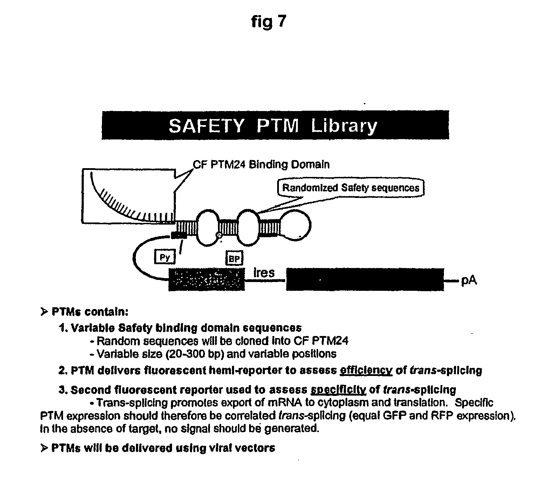

Figure 7 is a representation of a library of safety PTMs targeting CFTR intron 9. The safety PTM has the same backbone structure as described above. Model of prototype 3' safety PTM libraries. 3' PTMs containing (i) a single or variable binding domain sequences (ii) one or more randomized "safety" sequences that may be complementary to the target, PTM, or neither (iii) sequences encoding a portion of a reporter molecule (hemi-reporter) to assess efficiency of *trans*-splicing (iv) an internal ribosome entry site (IRES); and (v) sequences encoding a full-length reporter to assess the specificity of splicing. The library will contain many possible variants of sequences that may facilitate formation of stem loop, which will block formation of a spliceosome on the PTM's splice site to various degrees. Those "safety" stem loops which have the least effect on efficiency will produce the highest levels of the hemi-reporter (GFP in this example) in conjunction with the lowest (proportionate) expression of the full length PTM reporter(s) (RFP in this example).

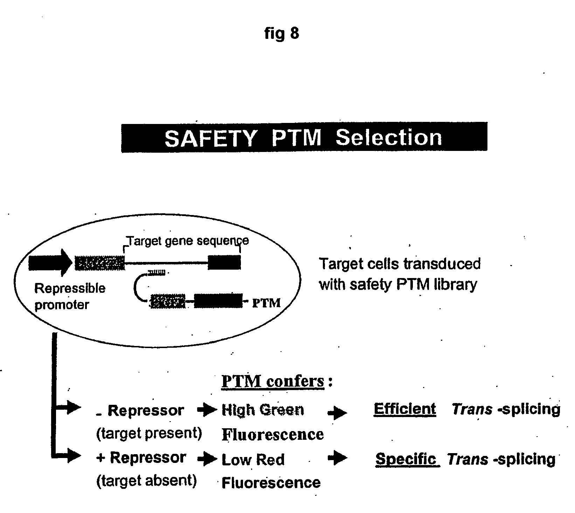

Figure 8 depicts the selection scheme for identifying optimal safety sequences. Target cells are transfected or transduced with a library of safety PTMs comprised of sequences encoding a portion of a first reporter molecule (e.g., a hemi- or partial green florescent protein, GFP), an IRES, and a second full-length reporter molecule (e.g., red florescent protein, RFP). The target cells express a target pre-mRNA that is under the control of an inducible promoter and that is comprised of sequences encoding the remaining portion of the first reporter (e.g., the region of the hemi-green florescent protein not encoded by the PTM), a target intron and a terminal exon(s). In the presence of the inducer, the target pre-mRNA is expressed. Specific *trans*-splicing of the candidate PTMs to the target pre-mRNA results in expression of the first reporter molecule (GFP), the level of which is indicative of the efficiency of *trans*-splicing. In the presence of target hemi-reporter pre-mRNA, expression of the repaired hemi-reporter (GFP) will be produced by specific *trans*-splicing, the level of GFP expression will indicate efficiency; expression of the PTM encoded full length reporter(s) (second reporter molecule, in this example RFP) driven off the IRES sequence will be the result of specific *trans*-splicing, as well as non-specific *trans-*splicing and direct PTM expression. An efficient PTM will produce high levels of the repaired hemi-reporter and low (proportionate) levels of the second full length reporter. A completely specific PTM should express a level of the second reporter that would be equivalent to the level of expression of the repaired hemi-reporter (taking into account any effect that the IRES has upon expression of the full-length reporter relative to the expression of the repaired hemi-reporter). In the absence of inducer, the target pre-mRNA is not expressed, and the level of expression of the second reporter (RFP) is correlated with the specificity of *trans*-splicing. In the absence of target pre-mRNA, the level of expression of the full length PTM encoded reporter will be the result of non-specific trans-splicing or direct PTM expression. This method can facilitate the identification of PTMs with desired or optimal specificity. Testing in the presence and absence of induction allows the assessment of the specificity of *trans*-splicing. GFP expression correlates with specific *trans-*splicing, the level of GFP expression will depend on the specificity of the PTM. For specific *trans*-splicing, GFP expression = yRFP expression, where y = the relative expression of IRES driven RFP expression compared to the expression of GFP in the same construct. Total RFP expression = yRFP + [non-specific trans-splicing RFP expression] + [direct PTM expression of the IRES-RFP].

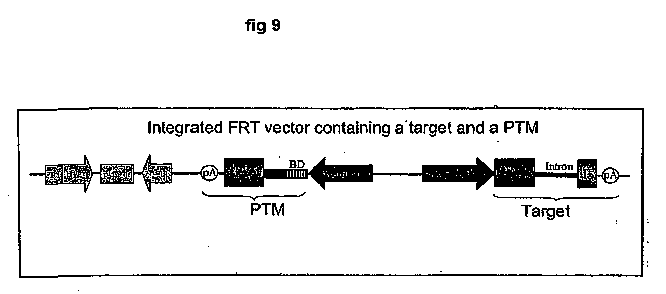

Figure 9. Depicts an integrated FRT vector containing a target and a PTM.

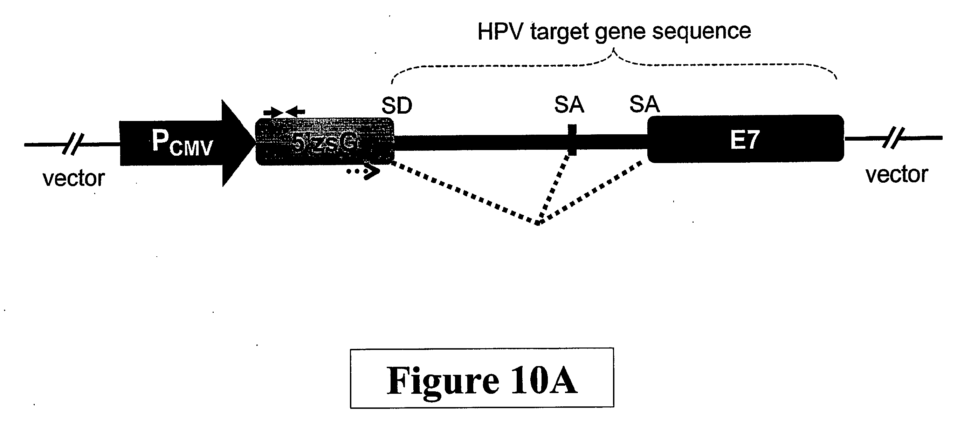

Figure 10A. Schematic diagram ofpre-mRNA target used in the high capacity screen. Abbreviations: SD, splice donor site; SA, splice acceptor site. Dotted lines indicate target cis-splice sites. Solid arrows indicate primer sites used for measuring cis-splicing; stripped arrow, forward primer used for measuring *trans-*splicing.

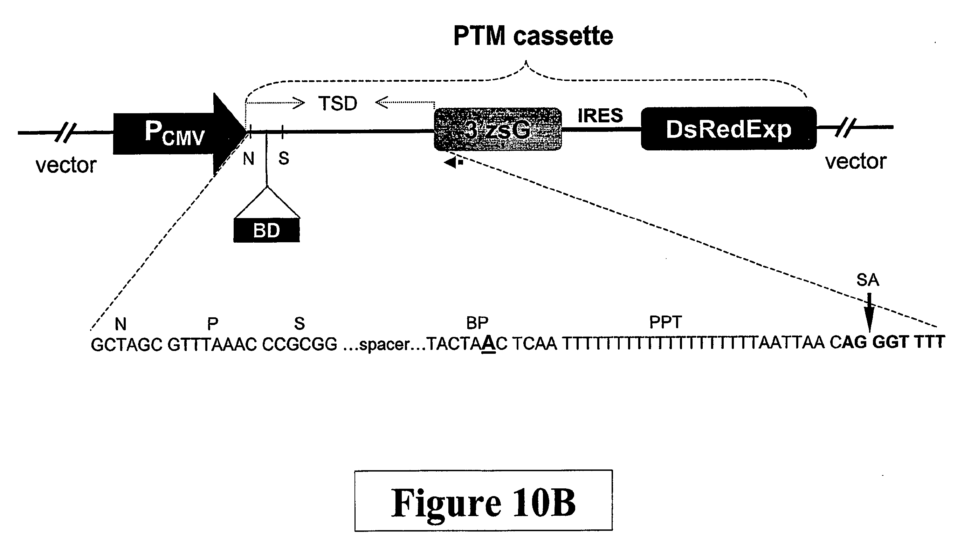

Figure 10B. Schematic illustration of PTM cassette used in the high capacity library screen. PTM cassette consists of a *trans*-splice domain (TSD) including: binding domain cloning sites, short spacer, BP, PPT, 3' half of the coding sequence for zsGreen, encephlomyocarditities (encephalomyocarditis virus) IRES followed by the full length coding sequence for second reporter, DsRedExpress. Abbreviations: 3'zsG, 3' half of the zsGreen fluorescent protein coding sequence; IRES, internal ribosome entry site, BD, binding domain; BP, branch point; PPT, polypyrimidine tract; N, *Nhe*I; P, *Pme*I, S, *Sac*II; and SA, splice acceptor site. Stripped arrow, reverse primer used for measuring *trans*-splicing efficiency.

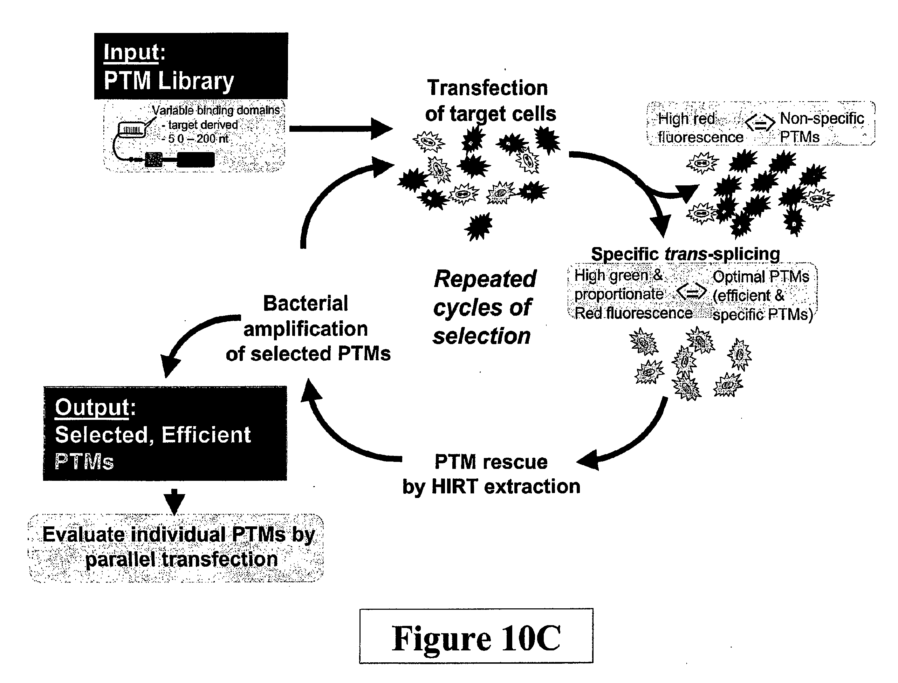

Figure 10C. Schematic illustration of the FACS-based selection strategy for PTMs directed toward the HPV-16 E6/E7 target pre-mRNA.

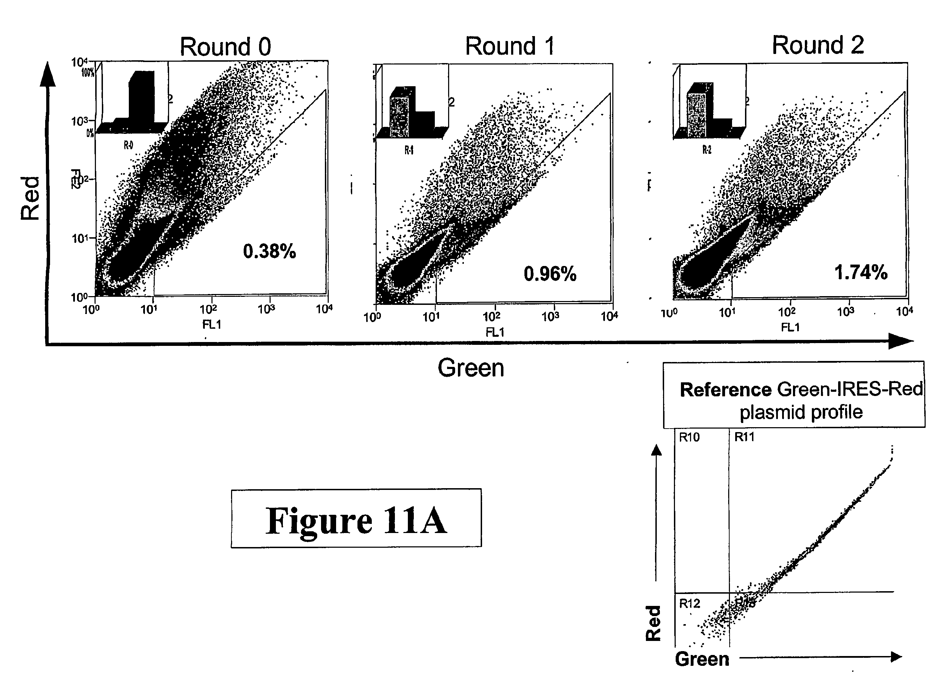

Figure 11A. Repeated rounds of selection enrich for *trans*-splicing. 293TE67 assay cells transfected with HPV PTM library using protoplasts. 24 hr post-transfection, cells were analyzed by FACS. Using the FACS profile of full-length green-IRES-DsRedexp as a reference (represents 100% *trans*-splicing efficiency and specificity), high green and proportionate red cells (gated region) from the library were collected, HIRT DNA extracted and used in the subsequent rounds of selection. Insert graphs show overall green/red ratio, an indication of the level of enrichment. REFERENCE: Full length GFP-IRES-RFP plasmid shows the FACS profile of a positive control plasmid construct in 293T cells. This control expresses the proportionate ratio of GFP/RFP expected from a completely specific (ideal) PTM trans-splicing of over a range of efficiency. This profile was used to set the gate for the PTM library screen to identify and select for optimal PTMs.

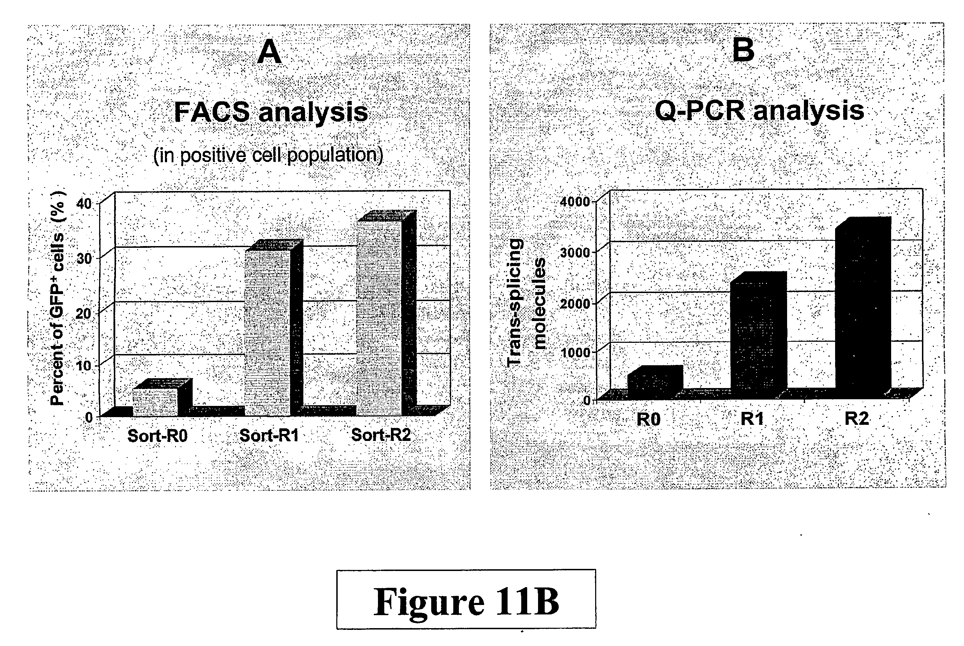

Figure 11B. Panel A, Repeated rounds of selection enrich for PTMs with improved trarrs-splicing. Experimental details are the same as described above. The percent of GFP⁺ cells from R0, R1 and R2 rounds are shown. The percentage of GFP⁺ cells are calculated using only the positive cell population. Similar results were also obtained using mean fluorescence values. Panel B. Repeated rounds of selection enrich for PTMs with improved *trans*-splicing. Experimental details are the same as described above. *Trans*-splicing efficiency at the molecular level was quantified by RT-qPCR of R0, R1 and R2 rounds. Total RNA from the total population (includes both positive and negative cells) was used to quantify *trans*-splicing efficiency by RT-qPCR. *Trans*-splicing efficiency was calculated by dividing the amount of *trans-*splicing by total splicing which includes both *cis-* and *trans*-splicing.

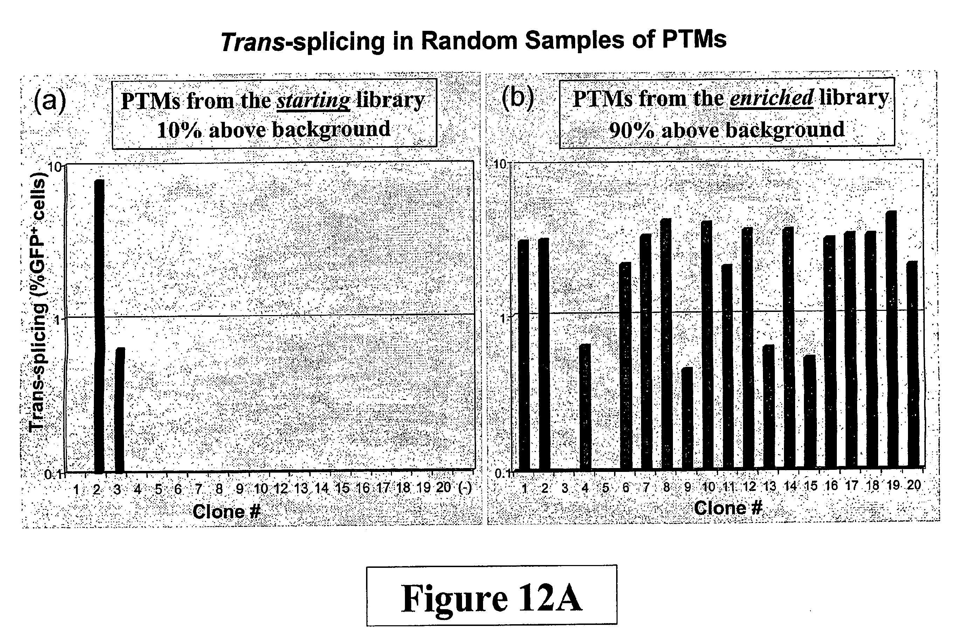

Figure 12A. High capacity screen enriches for efficient PTMs. DNA from 20 random PTMs of starting library and enriched library were transfected into 293TE67-12 stable cells. 24 hr post-transfection cells were analyzed by FACS for GFP expression. Panel a, clones from starting library, and panel b, clones from enriched library.

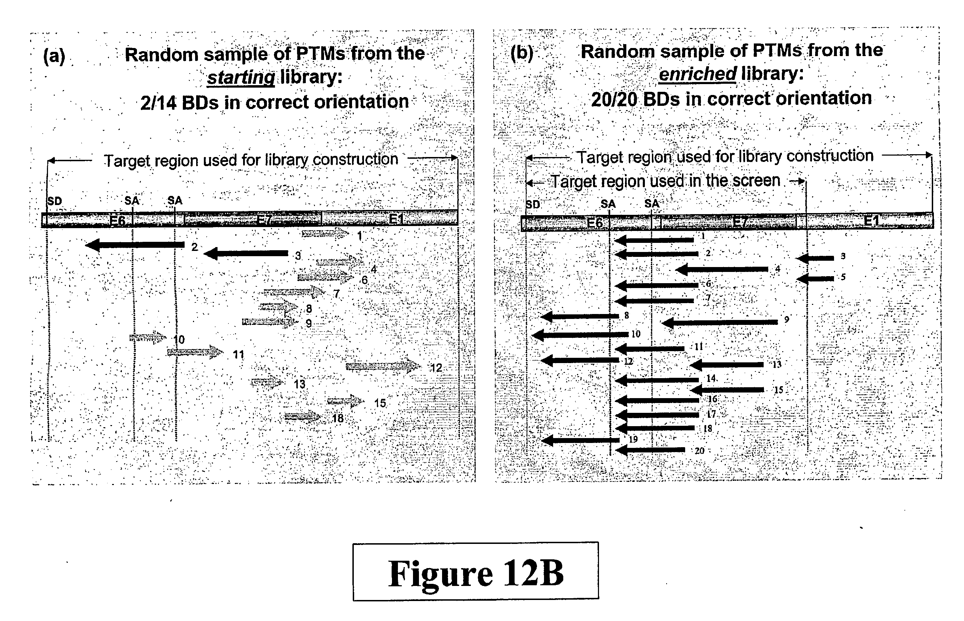

Figure 12B. Screen enriches for complementary binding domains. Comparison of BD size, orientation and positions of the individual libraries. Random clones from the original library (panel a), and enriched library (panel b) were sequenced and aligned against HPV target (HPV-16 E6/B7) used in the HCS. 2/14 are in correct orientation in the starting library, while, 100% are in correct orientation in the enriched library.

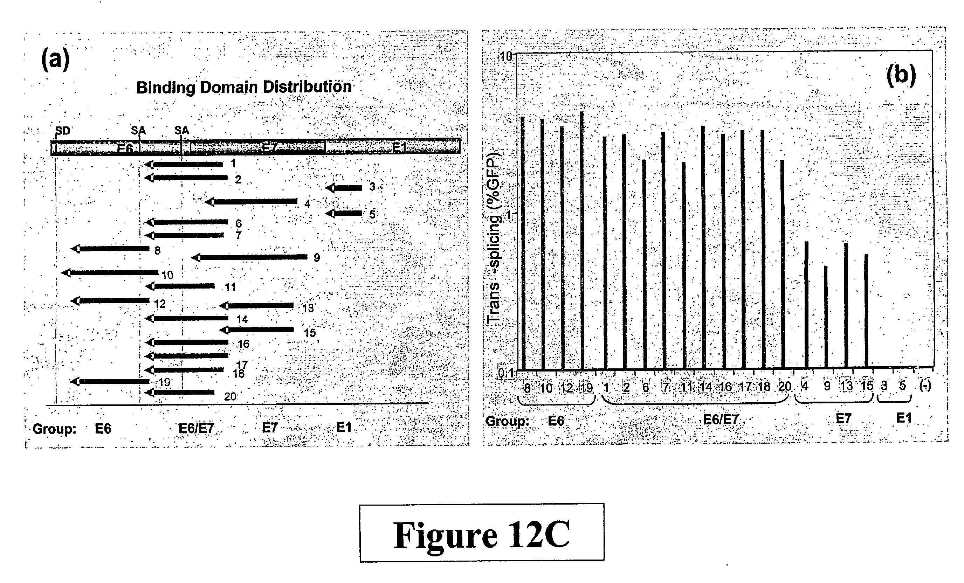

Figure 12C. PTMs recovered from HCS show hotspots of preferred target sites. Panel a, sequence alignment of PTMs from the enriched library against HPV target; panel b, FACS results from the same group. Sequence alignment revealed a good correlation between the position of the BDs vs. trans-splicing efficiency.

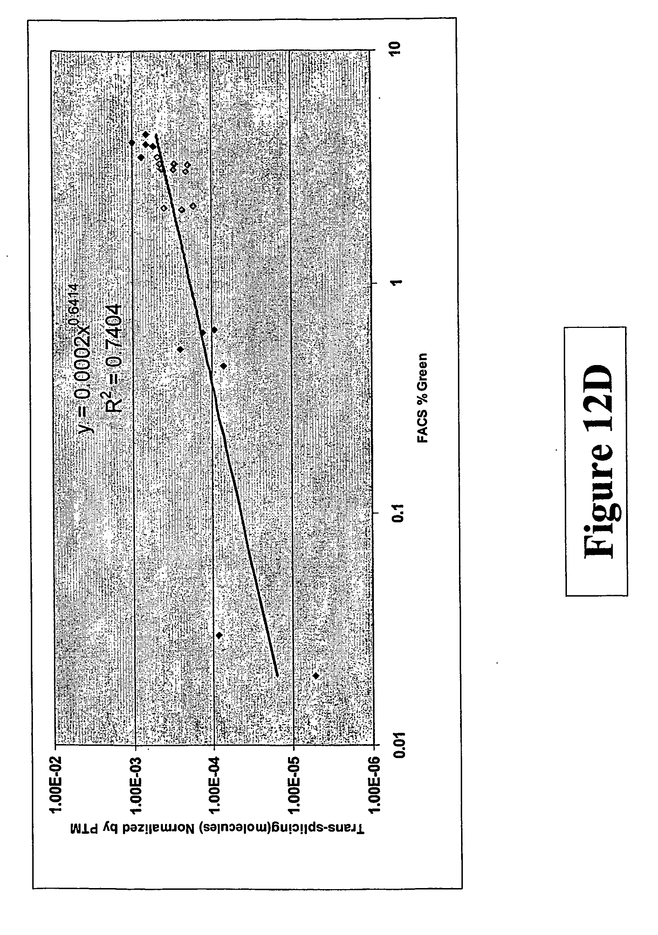

Figure 12D. PTMs recovered from the high capacity screen show a good correlation between function and molecular *trans*-splicing efficiency.

### 5. DETAILED DESCRIPTION OF THE INVENTION

The present invention provides methods and compositions for rapid high capacity functional screening for optimal pre-*trans*-splicing molecules. The compositions of the invention include PTM expression libraries capable of encoding candidate PTMs designed to interact with a target pre-mRNA and mediate a *trans-*splicing reaction resulting in the generation of a novel chimeric RNA molecule. As described in detail below, the compositions and screening methods of the invention can be used to rapidly evaluate, compare and identify optimal PTMs on the basis of their ability to mediate an efficient and specific *trans*-splicing reaction.

### 5.1. EXPRESSION LIBRARIES OF CANDIDATE PRE-TRANS-SPLICING MOLECULES

The present invention provides compositions for rapid high capacity functional screening for optimal PTMs. The compositions of the invention include PTM expression libraries capable of encoding candidate PTMs. In general, PTMs comprise one or more of the following structural elements: (i) one or more target binding domains that targets binding of the PTM to a pre-mRNA, (ii) a 3' splice region and/or 5' splice donor site, (iii) one or more spacer regions to separate the RNA splice site from the target binding domain, and (iv) a "safety sequence." The 3' splice region may include a branch point, pyrimidine tract and/or a 3' splice acceptor site. PTMs may also comprise mini introns, ISAR (intronic splicing activator and repressor) consensus binding sites, ribozyme sequences, and binding domains targeted to intron sequences in close proximity to the 3' splice signals of the target intron. The general design, construction and genetic engineering of such PTMs and demonstration of their ability to mediate successful *trans*-splicing reactions within the cell are described in detail in U.S. Patent Nos. 6,083,702, 6,013,487 and 6,280,978 as well as US patent applications Serial No. 09/941,492.

The candidate PTMs of the present invention are designed to include one or more of the structural elements normally associated with PTMs, however, at least one element is replaced with random or a multiplicity of nucleotide sequences. The random nucleotide sequences contain at least 15-30 and up to several hundred nucleotides depending on the element being replaced. The random nucleotides sequences for use in the candidate PTM molecules can be generated using a variety of different methods, including, but not limited to partial digestion of DNA with restriction endonucleases, mechanical shearing, sonication of the DNA or chemical synthesis. The use of such random nucleotide sequences is designed to generate a vast array of PTM molecules with different *trans*-splicing capabilities for any given target pre-mRNA expressed within a cell. Alternatively, randomized libraries of oligonucleotides can be synthesized with appropriate restriction endonucleases recognition sites on each end for cloning into PTM molecules. When the randomized oligonucleotides are litigated and expressed, a randomized library or candidate PTMs is generated.

In instances where the goal is to identity an optimal target binding domain for a specific target pre-mRNA, the random nucleotide sequences for use in the candidate PTM molecules can be generated using a variety of different methods, including, but not limited to, partial digestion of DNA encoding the target pre-mRNA with restriction endonucleases, or mechanical shearing or sonication of the DNA encoding the target pre-mRNA. Appropriate restriction endonucleases recognition sites can be cloned on each end of the random nucleotide sequences for cloning into PTM molecules.

In addition, the candidate PTM molecules of the invention are designed to express reporter molecules in the presence of an efficient *trans*-splicing reaction thereby providing a means for selection of cells expressing optimal PTMs. Thus, a nucleotide sequence encoding either a portion of a reporter molecule (hemi-reporter), or complete reporter molecule, is included in the candidate PTMs of the invention. Such reporter molecules include but are not limited to bioluminescent and fluorescent molecules, receptors, enzymes, and protein/peptide tags (Yu et al., 2000 Nature Medicine 6:933-937; MacLarent et al., 2000 Biol Psychiatry 48:337-348; Zaret et al., 2001 J. Nuclear Cardiology March/April 256-266; Ray et al., 2001 Seminars in Nuclear Medicine 31:312-320; Lok, 2001 Nature 412:372-374; Allport et al., 2001 Experimental Hematology 29:1237-1246; Berger and Gambhir, 2000 Breast Cancer Research 3:28-35; Cherry and Gambhir, 2001, ILAR Journal 42:219-232). Bioluminescent molecules include but are not limited to firefly, Renilla or bacterial luciferase. Fluorescent molecules include, for example, green fluorescent protein or red fluorescent protein.

In yet another embodiment of the invention, the reporter molecule may be an enzyme such as β-galactosidase (Louie et al., 2000 Nature Biotechnology 15:321-325), cytosine deaminase, herpes simplex virus type I thymidine kinase, creatine kinase (Yaghoubi et al., 2001 Human Imaging of Gene Expression 42:1225-1234; Yaghoubi et al., 2001 Gene Therapy 8:1072-1080; Iyer et al., 2001 J. Nuclear Medicine 42:96-105), or arginine kinase, to name a few. The enzyme is selected because of its ability to trap a specific radio labeled tracer by action of the enzyme on a chosen tracer.

Alternatively, the nucleotide sequences can encode for an extracellular marker protein, such as a receptor, which is capable of binding to a labeled tracer that has a binding affinity for the expressed marker protein. Such proteins include, for example, the dopamine 2 receptor, somatostatin receptor, oxotechnetate-binding fusion proteins, gastrin-releasing peptide receptor, cathepsin D, the transferrin receptor or the CFTR Cl⁻ ion channel.

In yet another embodiment of the invention, the reporter molecule may also be a protein or enzyme that confers resistance to an antibiotic, such as hygromycin or other selectable marker.

Nucleotide sequences encoding peptide tags, also referred to as epitope tags, may also be included in the structure of the PTMs of the invention to serve as reporter molecules. In a preferred embodiment of the invention, the epitope is one that is recognized by a specific antibody or binds to a specific ligand, each of which may be labeled, thereby providing a method for selection of cells expressing the target pre-mRNA. Eptiopes that may be used include, but are not limited to, AU1, AU5, BTag, c-myc, FLAG, Glu-Glu, HA, His6, HSV, HTTPHH, IRS, KT3, Protein C, STag, T7, V5, or VSV-G.

In addition, the candidate PTMs may further comprise an internal ribosome entry site (IRES), such as the encephalomyocarditis virus or poliovirus IRES followed by a second reporter molecule which can be used to evaluate the specificity of *trans*-splicing (see, for example, US Patent 4,937,190, W09811241 and Pestova TV et al. (2001, Proc Natl Acad Sci U S A.98:7029-36). The presence of IRES sequences followed by a second reporter molecule permits one to assess the specificity of *trans*-splicing. For example, the level of expression of the split hemi-reporter is a measure of specific *trans*-splicing. The level of expression of the PTM full length reporter(s) is the sum of specific and non-specific events. Thus the second reporter allows for the measurement of the extent of non-specific PTM expression. Since splicing promotes export of mRNA to the cytoplasm, levels of expression of the second reporter molecule in the absence of the pre-mRNA target should be inversely correlated with the specificity of the *trans*-splicing reaction.

To form the expression libraries of the invention, nucleic acid molecules encoding an array of candidate PTMs of interest are engineered into a variety of host vector systems that also provide for replication of the DNA in large scale and contain the necessary elements for directing the transcription of the candidate PTM in transfected or transduced cells. Methods commonly known in the art of recombinant DNA technology which can be used are described in Ausubel et al. (eds.), 1993, Current Protocols in Molecular Biology, John Wiley & Sons, NY; and Kriegler, 1990, Gene Transfer and Expression, A Laboratory Manual, Stockton Press, NY.

Vectors encoding the candidate PTMs can be plasmid, viral, or others known in the art, used for replication and expression in mammalian or other cell types, such as plant cells. Expression of the sequence encoding the PTM can be regulated by any promoter known in the art to act in the appropriate cell type, which may be mammalian, or preferably human cells. Such promoters can be inducible or constitutive. Such promoters include but are not limited to: the SV40 early promoter region (Benoist, C. and Chambon, P. 1981, Nature 290:304-310), the promoter contained in the 3' long terminal repeat ofRous sarcoma virus (Yamamoto et al., 1980, Cell 22:787-797), the herpes thymidine kinase promoter (Wagner et al., 1981, Proc. Natl. Acad. Sci. U.S.A. 78:14411445), the regulatory sequences of the metallothionein gene (Brinster et al., 1982, Nature 296:39-42), the viral CMV promoter, the human chorionic gonadotrophin-β promoter (Hollenberg et al., 1994, Mol. Cell. Endocrinology 106:111-119), etc. Any type of plasmid, cosmid, or viral vector can be used to prepare the recombinant DNA constructs which will form the PTM expression libraries of the invention.

The use of such constructs to transfect or transduce cells expressing the target pre-mRNA will result in the transcription of sufficient amounts of candidate PTMs wherein an optimal PTM will form complementary base pairs with the endogenously expressed target pre-mRNA and thereby facilitate a *trans*-splicing reaction between the complexed nucleic acid molecules.

The present invention further provides target cells recombinantly engineered to express a target pre-mRNA which can be used in the library screening methods of the invention. The target pre-mRNA may be transiently or stably introduced. For purposes of the present invention, it is important that the level of target pre-mRNA expression is equivalent in all cells evaluated. The target pre-mRNA is designed to serve as a substrate for *trans*-splicing in the presence of an optimal candidate PTM. Further, the target pre-mRNA is engineered to encode a portion of the hemi-reporter molecule (hemi-reporter target pre-mRNA) wherein a specific *trans*-splicing reaction results in a repaired chimeric mRNA capable of encoding a reporter molecule. In a preferred embodiment of the invention, the expression of the target pre-mRNA is under the control of an inducible promoter. Such inducible promoters which are well known to those of skill in the art, include for example, those promoters that respond to heat, steroid hormones, heavy metal ions and interferon. In addition, inducible promoter sequences may include those that utilize either the *E.coli* lactose (Lac) or the Tn10 derived tetracycline resistance operon responsive repressor elements. Inducible expression of the target pre-mRNA allows one to test the specificity of a *trans*-splicing reaction by comparing the level of observed reporter molecule in the presence and absence of target pre-mRNA.

Alternatively, nucleic acids encoding the target pre-mRNA may be recombinantly engineered into a gutless adenovirus-transposon vector which stably maintains virus encoded transgenes, i.e., the target pre-mRNA, in vivo through integration into the host cell chromosome (Yant et al., 2002, Nature Biotechnology 20:99-1005).

The expression cassettes of the hemi-reporter target or PTM, or both may be bounded by transcription insulator sequences, such as the chicken beta-globin insulator to minimize any position effects on transcription from outside regulator sequences. Recillas-Targa F, Pikaart MJ, Burgess-Beusse B, Bell AC, Litt MD, West AG, Gaszner M, Felsenfeld G.; Position-effect protection and enhancer blocking by the chicken beta-globin insulator are separable activities. Proc Natl Acad Sci USA. 2002 May 14;99(10):6883-8. Other insulator sequences are possible to use. A review on the subject: Genes Dev 2002 Feb 1;16(3):271-88. Insulators: many functions, many mechanisms. West AG, Gaszner M, Felsenfeld G.

In a specific embodiment of the invention, both PTM and pre-mRNA encoding sequences may be engineered into a single expression vector for transfection or transduction into the cell. The use of a single plasmid offers a more rapid method for screening a library of PTMs.

The present invention provides a novel selection system for identifying optimal PTMs based on their ability to mediate an efficient and specific *trans*-splicing reaction. The selection system of the invention comprises (i) a PTM expression library capable of encoding candidate PTMs, and (ii) a cell genetically engineered to express a target pre-mRNA. Both the PTMs of the expression library and the target pre-mRNA are designed to express a portion of a reporter molecule (hemi-reporter molecule) wherein in the presence of a specific *trans*-splicing reaction a repaired chimeric RNA capable of encoding a reporter molecule is formed.

In addition to *in vivo* sreening assays, the present invention also relates to *in vitro* screening methods designed to identify PTMS capable of mediating a *trans-*splicing reaction. In such instances, the *in vitro* assays are carried out in the presence of (i) a target pre-mRNA; (ii)one or more test PTMS; and a mixture of components necessary for spliceosome mediated trans-splicing. Such components may be derived from cell extracts.

In specific embodiments of the invention, the target pre-mRNA, the test PTM(s), or both, may be associated with a matrix. In addition, the target pre-mRNA, test PTM(s), or both, may be labeled in such a way as to easily identify successful *trans*-splicing reactions.

### 5.2 SCREENING OF PTM LIBRARIES FOR IDENTIFICATION OF OPTIMAL PTMS

The present invention provides screening methods which can be used to rapidly identify, evaluate, and compare PTMs on the basis of their ability to mediate a *trans*-splicing reaction with a target pre-mRNA. In a preferred embodiment of the invention, the screening methods of the invention may be used to identify optimal PTMs based on their ability to mediate a more efficient and/or specific *trans-*splicing reaction as compared to other PTM molecules also capable of mediating said *trans*-splicing reaction with a target pre-mRNA. The screening methods of the invention encompass (i) contacting a PTM library with target cells expressing a target pre-mRNA under conditions in which a *trans*-splicing reaction will occur in the presence of a PTM resulting in the formation of a chimeric RNA molecule capable of encoding a reporter molecule; (ii) selecting for cells expressing the reporter molecule wherein expression of the reporter molecule indicates the presence of an PTM capable of mediating a *trans*-splicing reaction in the selected cell; and (iii) identifying the said PTM expressed in the selected cells.

A variety of different methods may be used to transfer the PTM expression library into the cells expressing the target pre-mRNA of interest (herein referred to as "target cells"). Such methods include electroporation, lipofection, calcium phosphate or DEAE-Dextran mediated transfection, bacterial protoplast fusion or viral infection. In some instances the method of transfer includes the transfer of a reporter molecule (different from the reporter molecule(s) encoded by PTMs) to the cells. The target cells are then placed under selection to isolate those cells that have taken up and are expressing candidate PTMs.

Following transfer of the PTM library into test cells, the test cells are selected for those cells expressing a PTM encoded reporter molecule. The method of screening to be utilized for selection of cells expressing optimal PTMs will depend on the type of selectable reporter molecule encoded by the PTM. For example, when a fluorescent marker(s) is used the cells can be sorted using a fluorescent activated cell sorter (FACS). Alternatively, antibodies may be used to sort cells expressing polypeptide markers. Such cells may be sorted based on antibody affinity using methods such as panning or affinity chromatography. In instances where genes encoding enzymes which confer resistance to various drugs are used as selective markers, the ability to survive or grow in the presence of such a normally toxic drug can be used for selection. Other methods will be obvious to those skilled in the art.

The second reporter/selectable marker may be used to select cells based on the balanced expression of the second reporter molecule in proportion to the repaired hemi-reporter. Using this double selection process cells are selected based on high expression of the first reporter molecule and proportionate expression of the second reporter molecule. A preferred method to determine the proportionate level of expression between the *trans*-spliced hemi-reporter and full length reporter is to construct a reporter expression control vector comprising (i) an inducible promoter, (ii) a full length first reporter molecule, such as GFP, (iii) an IRES, and (iv) a full length second reporter, such as RFP. By measuring the level of, for example, GFP to RFP produced in cells, especially over a range of mRNA expression, the ratio of GFP to RFP expression can be determined. Thus, in any cell which expresses repaired (*trans*-spliced) GFP, a prediction can be made about the level of RFP expression that is expected by specific *trans*-splicing. In the context of the PTM library screen, any deviation above or below this level of RFP expression would result from non-specific *trans*-splicing, direct PTM expression, or a mutation in the PTM.

Following selection, clonal populations of the sorted cells are expanded for use in identifying the PTM expressed in the selected cell, *i.e.,* a lead candidate, possible optimal PTM. Alternatively, the cells may be selected a second time based on expression of a second (full length) reporter molecule in the absence of target. Nucleic acid sequences encoding the second (full length) reporter molecule are located downstream from the IRES sequences in the candidate 3' exon replacement PTMs. Expression of the second reporter molecule will occur in (i) the fraction of cells where correct *trans*-splicing has occurred; (ii) the fraction of cells where incorrect splicing has occurred; and (iii) cells where PTMs have been exported and self expressed. Thus, for the second selection protocol the level of specific trans-splicing is minimized due to the removal of the inducer, so that the level of target pre-mRNA is minimized and cells are selected based on the lowest level of expression of the second reporter molecule. Using this double selection process cells are selected based on high expression of the first reporter molecule and proportionate or low expression of the second reporter molecule, depending on the level of the target pre-mRNA present.

Once clonal populations of selected cells have been obtained, the PTMs are recovered and sequenced using routine methods. For example in a preferred embodiment, a polymerase chain reaction can be used to identify the lead binding domain using primers which flank the variable region. Alternatively, the PTM vector backbone can be recovered if it is an episomal plasmid, or by affinity hybridization. Trans-spliced RNA can be evaluated by reverse transcription and rapid amplification of cDNAs ends (5' RACE or 3'RACE) followed by cloning and sequencing of a statistically relevant sample of randomly selected clones.

### 5.3. USES OF PTMS IDENTIFIED BY SCREENING METHODS OF THE INVENTION

The optimal PTMs identified using the methods and compositions of the present invention will have a variety of different applications including gene repair, gene regulation and targeted cell death. For example, *trans*-splicing can be used to introduce a protein with toxic properties into a cell. In addition, PTMs can be engineered to bind to viral mRNA and destroy the function of the viral mRNA, or alternatively, to destroy any cell expressing the viral mRNA. In yet another embodiment of the invention, PTMs can be engineered to place a stop codon in a deleterious mRNA transcript, thereby, decreasing the expression of that transcript.

Targeted *trans*-splicing, including double-*trans*-splicing reactions, 3' exon replacement and/or 5' exon replacement can be used to repair or correct transcripts that are either truncated or contain point mutations. The PTMs of the invention are designed to induce a spliceosome to *trans*-splice a targeted transcript upstream or downstream of a specific mutation or upstream of a premature 3' stop codon and correct the mutant transcript replacing the portion of the transcript containing the mutation with a functional sequence.

Various delivery systems are known and can be used to transfer the compositions of the invention into cells, *e.g*. encapsulation in liposomes, microparticles, microcapsules, recombinant cells capable of expressing the composition, receptor-mediated endocytosis (see, *e.g*., Wu and Wu, 1987, J. Biol. Chem. 262:4429-4432), construction of a nucleic acid as part of a retroviral or other vector, injection of DNA, bacterial protoplast fusion electroporation, calcium phosphate mediated transfection, etc.

The compositions and methods can be used to treat cancer and other serious viral infections, autoimmune disorders, and other pathological conditions in which alteration or elimination of a specific cell type would be beneficial. Additionally, the compositions and methods can be used to provide a gene encoding a functional biologically active molecule to cells of an individual with an inherited genetic disorder where expression of the missing or mutant gene product produces a normal phenotype.

In a preferred embodiment, nucleic acids comprising a sequence encoding a PTM are administered to promote PTM function, by way of gene delivery and expression into a host cell. In this embodiment of the invention, the nucleic acid mediates an effect by promoting PTM production. Any of the methods for gene delivery into a host cell available in the art can be used according to the present invention. For general reviews of the methods of gene delivery see Strauss, M. and Barranger, J.A., 1997, Concepts in Gene Therapy, by Walter de Gruyter & Co., Berlin; Goldspiel et al., 1993, Clinical Pharmacy 12:488-505; Wu and Wu, 1991, Biotherapy 3:87-95; Tolstoshev, 1993, Ann. Rev. Pharmacol. Toxicol. 33:573-596; Mulligan, 1993, Science 260:926-932; and Morgan and Anderson, 1993, Ann. Rev. Biochem. 62:191-217; 1993, TIB TECH 11(5):155-215. Exemplary methods are described below.

Delivery of the nucleic acid into a host cell may be either direct, in which case the host is directly exposed to the nucleic acid or nucleic acid-carrying vector, or indirect, in which case, host cells are first transformed with the nucleic acid *in vitro,* then transplanted into the host. These two approaches are known, respectively, as *in vivo* or *ex vivo* gene delivery.

In a specific embodiment, the nucleic acid is directly administered *in vivo*, where it is expressed to produce the PTM. This can be accomplished by any of numerous methods known in the art, *e.g*., by constructing it as part of an appropriate nucleic acid expression vector and administering it so that it becomes intracellular, *e.g*. by infection using a defective or attenuated retroviral or other viral vector (see U.S. Patent No. 4,980,286), or by direct injection of naked DNA, or by use of microparticle bombardment (*e.g*., a gene gun; Biolistic, Dupont), or coating with lipids or cell-surface receptors or transfecting agents, encapsulation in liposomes, microparticles, or microcapsules, or by administering it in linkage to a peptide which is known to enter the nucleus, by administering it in linkage to a ligand subject to receptor-mediated endocytosis (see *e.g*., Wu and Wu, 1987, J. Biol. Chem. 262:4429-4432).

In a specific embodiment, a viral vector that contains the PTM can be used. For example, a retroviral vector can be utilized that has been modified to delete retroviral sequences that are not necessary for packaging of the viral genome. (see Miller et al., 1993, Meth. Enzymol. 217:581-599). Alternatively, adenoviral or adeno-associated viral vectors can be used for gene delivery to cells or tissues. (See, Kozarsky and Wilson, 1993, Current Opinion in Genetics and Development 3:499-503 for a review of adenovirus-based gene delivery).

Another approach to gene delivery into a cell involves transferring a gene to cells in tissue culture by such methods as electroporation, bacterial protoplast fusion, lipofection, calcium phosphate mediated transfection, or viral infection. Usually, the method of transfer includes the transfer of a reporter molecule to the cells. The cells are then placed under selection to isolate those cells that have taken up and are expressing the transferred gene. The resulting recombinant cells can be delivered to a host by various methods known in the art. In a preferred embodiment, the cell used for gene delivery is autologous to the host cell. Integration of the PTMs can be accomplished by incorporating specific integration sites such as Flp recombination or Cre-Lox sites into the target cell line or the PTM vector itself.

The present invention also provides for pharmaceutical compositions comprising an effective amount of a PTM or a nucleic acid encoding a PTM, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Examples of suitable pharmaceutical carriers are described in "Remington's Pharmaceutical sciences" by E.W. Martin.

In specific embodiments, pharmaceutical compositions are administered: (1) in diseases or disorders involving an absence or decreased (relative to normal or desired) level of an endogenous protein or function, for example, in hosts where the protein is lacking, genetically defective, biologically inactive or underactive, or under expressed; or (2) in diseases or disorders wherein, *in vitro* or *in vivo,* assays indicate the utility of PTMs that inhibit the function of a particular protein. The activity of the protein encoded for by the chimeric mRNA resulting from the PTM mediated *trans*-splicing reaction can be readily detected, *e.g*., by obtaining a host tissue sample (*e.g*., from biopsy tissue) and assaying it *in vitro* for mRNA or protein levels, structure and/or activity of the expressed chimeric mRNA. Many methods standard in the art can be thus employed, including but not limited to immunoassays to detect and/or visualize the protein encoded for by the chimeric mRNA (*e.g*., Western blot, immunoprecipitation followed by sodium dodecyl sulfate polyacrylamide gel electrophoresis, immunocytochemistry, etc.) and/or hybridization assays to detect formation of chimeric mRNA expression by detecting and/or visualizing the presence of chimeric mRNA (*e.g*., Northern assays, dot blots, in situ hybridization, and Reverse-Transcription PCR, etc.), etc.

The present invention also provides for pharmaceutical compositions comprising an effective amount of a PTM or a nucleic acid encoding a PTM, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Examples of suitable pharmaceutical carriers are described in "Remington's Pharmaceutical sciences" by E.W. Martin. In a specific embodiment, it may be desirable to administer the pharmaceutical compositions of the invention locally to the area in need of treatment. This may be achieved by, for example, and not by way of limitation, local infusion during surgery, topical application, *e.g*., in conjunction with a wound dressing after surgery, by injection, by means of a catheter, by means of a suppository, or by means of an implant, said implant being of a porous, non-porous, or gelatinous material, including membranes, such as sialastic membranes, or fibers. Other control release drug delivery systems, such as nanoparticles, matrices such as controlled-release polymers, hydrogels.

The PTM will be administered in amounts which are effective to produce the desired effect in the targeted cell. Effective dosages of the PTMs can be determined through procedures well known to those in the art which address such parameters as biological half-life, bioavailability and toxicity. The amount of the composition of the invention which will be effective will depend on the nature of the disease or disorder being treated, and can be determined by standard clinical techniques. In addition, *in vitro* assays may optionally be employed to help identify optimal dosage ranges.

### 6. EXAMPLE: SELECTION FOR OPTIMAL TRANS-SPLICING PTM MOLECULES THAT REPAIR THE CYSTIC FIBROSIS TRANSMEMBRANE REGULATOR (CFTR) mRNA

The library screening method described herein can be used to select for CFTR based PTMs with improved function. In addition to binding domains, other structural elements within PTM libraries can be replaced with random nucleotide sequences to generate a vast array of candidate PTMs with different *trans*-splicing capabilities. One such element, safety sequences, are capable of forming secondary structures within the PTM molecule whereby the splicing elements in the PTM are blocked from splicing until the binding domain interacts with the target pre-mRNA. CFTR based PTMs with improved function can be identified by (i) improving the specificity of *trans*-splicing by screening libraries of randomized "safety" PTM binding domains based on an existing PTM, CF PTM24, and selecting those which are most efficient and specific in repairing the expression of fluorescent reporters; and (ii) quantifying the specificity of lead safety PTMs by sequencing cloned unselected *trans*-spliced mRNA products. This method will result in the identification of a PTM with significantly improved performance over the currently existing CF targeted PTM 24 and improve the probability for the successful development of a clinical therapeutic agent.

To date, over 30 CF targeted 3' PTMs have been empirically constructed which differ from each other primarily in the length and region of base pairing to the CFTR pre-mRNA. The *trans*-splicing efficiency of the existing PTMs varies considerably. Since cells in culture do not normally express CFTR, a CFTR intron 9 target was constructed to study *trans*-splicing of the test PTMs. This target consists of 250 nucleotides of the 5' end and 270 nucleotides from the 3' end of intron 9 and has been very useful for rapidly evaluating the efficiency of PTMs constructed to date.

New PTMs identified by the high throughput screen described herein will be evaluated for their capacity to restore CFTR function. This will permit selection of optimal CF targeted PTMs that will be evaluated for potential to enter pre-clinical and ultimately human clinical trials.

### 6.1. LIBRARY SCREEN

The novel library screening assay described herein is designed to rapidly select PTMs which are optimal in terms of efficiency and specificity. The assay is based on the power of FACS sorting to evaluate tens of millions of cells per hour and collect those few cells with desired characteristics. In this assay, each transduced cell becomes a separate experiment used to evaluate the efficiency and specificity of a unique PTM based on the readout of two fluorescent markers. This permits rapid screening of millions of PTMs in a cost efficient manner and selection of lead PTMs which can be further characterized to identify the best PTM for entry into a clinical development path.

### 6.2. SAFETY PTM LIBRARIES

A library of safety PTMs targeting CFTR intron 9 is constructed by incorporating randomized sequences adjacent to the binding domain of CF PTM 24 to produce safety stems ranging from 10 to 300 bp in length. It is believed that stem loops in this size range will be optimal, based on previous experiments with empirically designed safety PTMs. These random sequences are cloned into a CF PTM24 expression cassette that will include a CMV promoter followed by the binding domain of CF PTM24, a randomized safety sequence, a spacer region, a strong 3' splice site (including a yeast consensus branch point, a strong polypyrimidine tract and splice acceptor site), and the 3' coding sequence of enhanced green fluorescent protein (eGFP) (Figure 7). Downstream of eGFP will follow an internal ribosome entry site (IRES) which directs the expression of red fluorescent protein for any PTM which reaches the cytoplasm and can be translated. Specific *trans*-splicing will produce levels of both green and red fluorescence. Non-specific *trans*-splicing, PTM *cis*-splicing or direct PTM translation will contribute only red fluorescence. Optimal PTMs are selected on the basis of high green/proportionate red signal as described below. It is anticipated that the library generated will have a complexity in the range of 10⁵ to 10⁶ independent safety PTMs.

### 6.3. PRODUCTION OF TARGET CELLS

A target cell line is constructed which contains a 5' hemi-eGFP gene coupled to a portion of CF intron 9 (Figure 8). The target cell line will contain the mini-intron 9 target sequence which has been previously employed to evaluate the function of previously constructed PTMs. A target expression vector comprising the 5' half of the coding sequence of enhanced green fluorescent protein (eGFP) and the CFTR mini-intron is stably integrated into 293 cells. A clonal cell line is established that produces consistent levels of CFTR target in each cell in the population. This is important as the efficiency of trans-splicing can vary with the concentration of both target pre-mRNA and PTM. The assay is constructed to minimize these variables.

The expression of target pre-mRNA is under the control of a tetracycline repressor. Cells will undergo FACS sorting in the absence of tet (first pass) under the condition of high target concentration. Selected cells are then grown in the presence of tet to repress target expression. This substantially reduces the concentration of target pre-mRNA and permits the evaluation of non-specific PTM expression due to trans-splicing to non-target pre-mRNAs or direct translation of the PTM itself.

### 6.4. DELIVERY OF PTM LIBRARY TO TARGET CELLS

Retroviruses can be used to deliver the PTM library. This method is useful because of its ability to deliver a single member of the PTM library to a single target cell. The retroviruses are designed to contain insulator sequences from chicken beta-globin to minimize the possible effects on transcription due to the random nature of retroviral integration. It is crucial to maintain consistent levels of expression for both the target and PTM in each cell in order to reliably utilize reporter gene expression for comparison of the specificity and efficiency of *trans*-splicing. If the concentration of target PTM varies between different cells readout could be substantially affected.

The safety PTM fluorescent reporter library is transfected into a retroviral production cell line to generate self-inactivating (SIN) murine retroviruses. These viruses will contain insulator sequences to minimize the possible effect on transcription due to the site of integration. The retroviruses are used to transduce 293 cells. The resultant cells are then FACS sorted.

### 6.5. SCREENING THE SAFETY PTM LIBRARY FOR THE MOST EFFICIENT AND SPECIFIC TRANS-SPLICING

The PTM safety library is transfected into the target cell line produced as described above. Specific *trans*-splicing of a PTM to its target will generate the complete coding sequence of eGFP. RFP expression is produced by the combination of all (i) specific *trans*-splicing plus non-specific events including (ii) *trans*-splicing into incorrect pre-mRNAs (in any reading frame) and (iii) direct translation of the PTM. Lead candidate safety PTMs are selected to maximize specific *trans*-splicing (high green) and to minimize non-specific expression of the PTM (proportionate red/green ratio). FACS sorting is performed to identify and select those cells transduced with PTMs that express maximal levels of eGFP and the proportionate level of RFP (one GFP should also produce 1 RFP). These cells are collected by the FACS instrument either as a mixed population or individually (such as into 96 well plates) containing the appropriate growth media. The lead safety PTM candidates are compared to each other and CF PTM24 (which is also to be included in the screen) to identify one or several lead safety CF PTMs to be further characterized. The second round of screening for specificity is performed on the collected mixed population or clonal sorted cells grown in the presence of tetracycline. Under this condition, the tet repressor will markedly reduce the production of the GFP-CFTR target. This permits the quantification of RFP expression in the absence of CFTR target. In the first round screen it may be difficult to distinguish the most efficient and specific PTMs from those which are equally efficient but produce slightly more non-specific RFP signal generated by inappropriate PTM expression if the RFP signal generated by correct *trans*-splicing is reduced or eliminated.

### 6.6. QUANTIFYING THE SPECIFICITY OF TRANS-SPLICING

The specificity of the lead safety PTMs is quantified as follows to select the final lead PTM for functional testing in phase II. Total RNA from ten FACS selected cell lines are isolated from cells grown without tet (CFTR target is present). This RNA is used in a 5' RACE procedure using a commercially available kit (Ambion). The resultant cDNA is the cloned. Ninety-six independent clones derived from unselected trans-spliced products generated by 5' RACE from each lead PTM are sequenced and the number of specific vs. non-specific trans-spliced events is quantified. The specificity of PTM 24 is also quantified by this method. The most specific of the PTMs is then evaluated for its ability to restore CFTR function in phase II, where the reporter molecules are replaced with exons encoding the portion of CFTR to be repaired. These PTMs will be tested in the context of cells derived from CF patients for their ability to correct the mutation and restore function in these cells, described in Liu, X, Q. Jiang, S.G. Mansfield, M. Puttaraju, Y. Zhang, W. Zhou, M. A. Garcia-Blanco, L.G. Mitchell and J.F. Engelhardt. Functional Restoration of CFTR Chloride Conductance in Human CF Epithelia by Spliceosome-mediated RNA Trans-splicing. Nature Biotechnology 20(1): 47-52, 2002.

### 7. EXAMPLE: SINGLE PLASMID VECTOR ENCODING A TARGET PRE-mRNA AND PTMS

### 7.1. VECTOR DESCRIPTION

The vector backbone consists of pcDNA5-FRT/TO for site specific integration or pcDNA5-TO for random integration (Invitrogen). Each vector has been re-engineered to transcribe a PTM and a target pre-mRNA in opposite directions using a different promoter/enhancer for each. Transcription level of the target is controlled by the Tet-on/ Tet-off inducible promoter present in the vector, while PTM transcription is controlled by a different, continuously active promoter. The latter would be carefully chosen to reduce interference between the target and the PTM promoters.

### 7.2. CLONING DETAILS

Sequences encoding the target pre-mRNA are cloned upstream of the CMV inducible promoter. The PTM cassette containing all elements but the binding domain is cloned at a different location under the control of a different promoter. This construct is then linearized at the cloning site for the PTM binding domain and a library of vectors are constructed by insertion of different sequences. The plasmids are then transfected into a 293 Flp-In T-Rex cell line (Invitrogen) to obtain a single integrated copy of a PTM and target at the endogenous FRT site (see Figure 9) of the cell line or at a random site.

### 8. EXAMPLE: SELECTION FOR OPTIMAL TRANS-SPLICING PTM MOLECULES BASED ON THE HBV-16 E6/E7 TARGET PRE-mRNA

As described below, the library screening method of the invention can be used to select for human papilloma virus (HPV) based PTMs with improved function. The high capacity screen takes advantage of the power of fluorescence activated cell sorting (FACS) in combination with repeated cycles of selection to rapidly evaluate millions of different sequence combinations and identify optimal PTMs. This approach is based on a hemi-reporter. However, this design also includes a second reporter that allows the simultaneous evaluation of *trans*-splicing specificity in a single screen. Two main components of the high capacity screen are: (i) a cell line (assay cells) that expresses the intended pre-mRNA target and (ii) a PTM library.

### 8.1 ASSAY CELLS

A stable target cell line was established that expresses the 5' half of the coding sequence for the green fluorescent protein (GFP) ("zsGreen" from Clontech, Palo Alto, Ca.) coupled to the non-coding sequence upstream of the human papilloma virus type 16 E6/E7 (HPV-16) gene. The target pre-mRNA contains nucleotides 1-210 of zsGreen coding sequence including the initiating ATG codon followed by nucleotides 226 through 880 of the HPV 16 E6/E7 gene (Figure 10A). Analysis of total RNA from cells transfected with the target plasmid, (pc5'GE67), by reverse transcription (RT) PCR produced the expected cis-spliced products but no GFP function was detected. Upon confirming the splicing pattern of the GFP-HPV 16 E6/E7 pre-mRNA target, a stable cell line in 293T cells was established by transfecting the target plasmid followed by hygromycin selection. Several individual clones were isolated and characterized by RT-PCR. Based on the results, a single clone (293TE67-12) that showed the highest target pre-mRNA expression was selected for the high capacity screen. Trans-splicing is facilitated in the presence of higher target concentrations

### 8.2 PTM CASSETTE

A schematic illustration of the PTM cassette used in the high capacity screen for PTMs directed toward the HBV-16 E6/E7 target pre-mRNA is shown in Figure 10B. The PTM cassette consists of a *trans*-splicing domain (TSD) that includes a unique *Pme*I restriction site for cloning randomized binding domains (BDs). Adjacent to the *Pme*I site there are unique *Nhe*I and *Sac*II sites that can be used to extract any lead BD for further testing. This is followed by a 24 nucleotide spacer region, a strong 3' splice site including the consensus yeast branch point (BP), an extended polypyrimidine tract (19 nucleotides long), a splice acceptor site (CAG dinucleotide), the remaining 3' coding sequence of zsGreen fluorescent protein (487 nucleotides) absent from the HBV-16 E6/E7 target pre-mRNA, followed by an encephalomyocarditis virus (ECMV) internal ribosomal entry site (IRES) and the complete coding sequence for DsRedExpress fluorescent protein (Clontech). The latter is used for the evaluation of *trans*-splicing specificity. Specific *trans*-splicing between the intended pre-mRNA target and the PTM produces both green and a proportionate amount of red fluorescence. Non-specific *trans*-splicing to non-target mRNAs or the direct translation of the PTM produces only red fluorescence.

### 8.3 CONSTRUCTION AND DELIVERY OF THE PTM LIBRARY

The HPV-16 E6/E7 gene sequence (from nt 79 through 1071, total of 992 bp) was fragmented into small pieces by sonication and fractionated on a 3% agarose gel. Fragments ranging in size from 50-250 nucleotides were gel purified and eluted. Fragment ends were repaired using Klenow enzyme and cloned into the PTM cassette described above (Figure 10B). PCR analysis of the library colonies showed >95% recombination efficiency and produced a library of up to 10⁶ independent clones with BDs varying in size from 50-250 nt. The primary library was amplified in bacteria and used for screening PTMs. The PTM library was delivered into assay cells using bacterial protoplasts. It is important to keep target expression relatively constant between cells. Varying target concentration between cells or delivering >1 PTM library member to a cell can affect the ability to efficiently estimate the level of trans-splicing between different cells.

### 8.4 FACS-BASED PTM SELECTION STRATEGY

The FACS-based selection strategy for PTMs directed toward the HBV-16 E6/E7 target pre-mRNA is illustrated in Figure 10C. First, the PTM library was transfected into assay cells expressing the pre-mRNA target. After 24 hrs, cells were analyzed for green fluorescent protein (GFP) expression using FACS. Specific *trans*-splicing between the pre-mRNA target and a PTM will result in the expression of GFP and a proportionate level of red fluorescent protein (RFP). The intensity of the green fluorescence is used as a measure of specific *trans*-splicing efficiency. Non-specific *trans*-splicing of PTMs into the wrong target pre-mRNAs, or direct expression of PTMs will cause the expression of RFP but no GFP. The difference in the expression pattern (i.e., high GFP and proportionate RFP vs. high RFP expression) was exploited for the selection of the lead PTMs in the screen. As illustrated in Figure 10C, cells expressing high GFP and proportionate RFP were collected; whereas, cells expressing high RFP were eliminated. A positive control plasmid expressing full length GFP-IRES-full length RFP was used as a reference to determine the region (gate) for selecting high green and proportionate red cells. The positive control represents an ideal PTM, with 100% *trans*-splicing efficiency and no non-specific activity. Using the control PTM FACS profile as a reference, lead PTMs were collected. The PTMs were rescued by HIRT DNA extraction followed by *Dpn* I treatment to eliminate the input library PTMs. The enriched PTM library from round 1 was amplified in bacteria, protoplasts were prepared and used for transfection of target cells in the next round of screening. This cycle of transfection followed by FACS selection was repeated until the ratio of green/red was sufficiently improved. The main purpose of repeating the selection process was to minimize and/or eliminate noise and false positives. For example, if the high GFP expression is due to the presence of >1 PTM library member per cell or variation in target expression, with repeated cycles of selection the false positives should be eliminated in subsequent rounds. Finally, once the desired green/red ratio is achieved, individual clones were isolated and assessed for *trans*-splicing specificity and efficiency.

### 8.5 RESULTS: REPEATED ROUNDS OF SELECTION ENRICH FOR TRANS-SPLICING

The HPV PTM-BD library was tested using the assay cells expressing the hemi-GFP-HPV-16 E6/E7 pre-mRNA target. Figures 11A-B show the results from two rounds of selection which produced approximately a 5-7 fold improvement at the functional level after two rounds of enrichment (R2) compared to the starting library (R0). The percent GFP⁺ cells were calculated taking into account only the positive cell population (Figure 11B). Similar results were also obtained using mean fluorescence values as the end point. As expected, >99% of the PTM library produced robust RFP expression and little or no GFP in R0 indicating that the majority of the BDs in the library were either (a) inefficient *trans*-splicers, (b) in the wrong orientation, (c) *trans*-spliced non-specifically to non-target pre-mRNAs, or (d) were directly expressed, for example, through cis-splicing within the PTM BD or vector backbone and PTM acceptor site. In addition to the enrichment at the functional level, we have also observed a significant reversal in the green/red ratio from R0 compared to R2, i.e., a green/red ratio of 0.1 was obtained for the starting library compared to 4.9 for the enriched library (Figure 11A inserts). In R0, even though several GFP expressing cells are seen, very high RFP expression was detected in most cells. However, after two rounds of selection a completely different pattern was observed in R2, i.e., more cells expressing proportionate levels of GFP/RFP and higher GFP were observed (Figure. 11B). This observation was confirmed at the molecular level by reverse transcription (RT) real time quantitative PCR (RT-qPCR) analysis to measure the efficiency of specific *trans*-splicing (restoration of GFP coding sequence). RNA from the total cell population (which includes both GFP⁺ and negative cells) was isolated and the amount of full length GFP mRNA was quantified by RT-qPCR. Target and PTM specific primers were used for measuring specific *trans*-splicing. Total *cis-, trans-,* and un-spliced target RNA was measured using primers specific for the 5'zsGreen exon (Figures 10A-B). Based on the qPCR result, a ∼6-7 fold improvement in *trans*-splicing efficiency was detected in the enriched library (R2) compared to the starting library (R0) (Figure 11B). The qPCR results are in close agreement with the FACS (functional) results

### 8.6 ENRICHED PTMs ARE SUPERIOR TRANS-SPLICERS

The results of repeated selection demonstrated a significant improvement in *trans*-splicing efficiency at the population level. Splicing efficiency was also tested for individual PTMs. Twenty random PTMs from the enriched library (R2) and 20 random PTMs from the starting library (R0) were selected for further testing by parallel transfection. Stable cells expressing the GFP- HBV-16 E6/E7 target pre-mRNA were transfected with the selected PTMs isolated from these libraries. At 48 hr post-transfection, cells were analyzed for GFP expression by FACS. As predicted, >90% of the PTMs from the enriched library showed *trans*-splicing activity above background compared to only 10% in the starting library (Figure 12A). These results were further confirmed at the molecular level. Total RNA from cells transfected with individual PTMs was isolated and the *trans*-splicing efficiency was measured by RT-qPCR as described above. The qPCR results were normalized for transfection efficiency by quantifying PTM expression. After correcting for transfection efficiency, qPCR results demonstrated levels of *trans*-splicing to be similar to the FACS results (production of GFP = function). PTMs that showed higher *trans*-splicing efficiency at the functional level were also more efficient in *trans*-splicing at the molecular level. This was true for PTMs from both the starting and enriched libraries.

Tests were done to determine whether the BD orientation and position of the PTM binding domain had any effect on trans-splicing efficiency and specificity. When sequences of random PTMs from the starting PTM library were compared to the enriched library, a surprising pattern was revealed with respect to BD orientation and position vs. *trans*-splicing. The results are summarized in Figure 12B. Sequence analysis also revealed why only 10% of the starting library PTMs were GFP+ compared to 90% in the enriched library. These data correlated with sequence analysis showing that 100% of the BDs from the enriched library were in the correct (antisense) orientation, compared to only 14% (2 out of 14) in the starting library. Furthermore, the mean BD length was significantly greater than in the starting library (164 vs 105 nucleotides), which is consistent with previous work demonstrating that certain length BDs are more efficient (Puttaraju *et al.,* 2001). In addition, sequence alignment revealed a correlation between the positions of the BDs vs. *trans*-splicing efficiency. Based on these results, the BDs from the enriched library could be grouped into 4 sub groups: (i) BDs located closest to the donor site, (ii) BDs blocking the 3' splice site at nucleotide 526 and spanning across the E6/E7 region, (iii) BDs downstream of the 3' splice site and binding mainly to the E7 region, and (iv) BDs that bind the E1 region in the target pre-mRNA (Figure 12C). PTMs with highest *trans*-splicing efficiency from both the starting library and enriched library were positioned in and around the E6/E7 region of the target, while PTMs with BDs that were positioned a distance from this region showed poor *trans*-splicing efficiency. These results indicate that there may be *"hotspots"* that are more accessible binding sites within the HPV-16 E6/B7 target pre-mRNA. Results of the selected (R2) PTMs also demonstrated a good correlation between molecular *trans*-splicing and function (Figure 12D) suggesting that the selection criteria used on the FACS to identify the lead PTMs were valid.

### 8.6 BDs ISOLATED FROM THE HIGH CAPACITY SCREEN SHOW ENHANCED TRANS-SPLICING SPECIFICITY.

The present invention provides a high capacity screen as a tool to isolate optimal PTMs with high *trans*-splicing specificity and efficiency. The specificity of *trans*-splicing was quantified by constructing a library of *trans*-spliced molecules using a 5'RACE technique followed by sequence analysis. The number of specific vs. non-specific *trans*-spliced mRNAs was qantified. To evaluate *trans*-splicing specificity, total RNA was extracted from cells collected from the enriched fraction (R2) that produces proportionate GFP/RFP and from the far-red region (containing mostly high RFP expressing cells) and used for 5'RACE library construction. Preliminary PCR results of screening ninety-six independent clones for specific *trans-*splicing showed at least a 10-fold enhancement in *trans*-splicing specificity in the enriched library compared to a library constructed using cells expressing high RFP. These results suggest that the IRES driven second reporter may be valuable in selecting highly specific and efficient PTMs. In addition, the 5'RACE results indicate that the repeated rounds of selection of the library also reduced or eliminated the number of un-spliced PTMs in the final library. Finally, the results presented here clearly demonstrate that high capacity screening of a high complexity PTM library is effective in identifying potential lead PTMs.

## Claims

1. A method for identifying optimal pre-trans-splicing molecules (PTMs) capable of interacting with a target pre-mRNA and of mediating a trans-splicing reaction therewith, resulting in the generation of a chimeric RNA molecule, the method comprising:
(I) contacting an expression library of candidate PTMs with cells expressing a target pre-mRNA under conditions in which such a trans-splicing reaction will occur in the presence of one or more PTMs, wherein said candidate PTMs comprise (i) a target-binding domain; (ii) a 3'-splice, region and/or a 5'-splice region; (iii) at least one nucleic acid sequence encoding a portion of a first reporter molecule, so that a successful trans-splicing reaction will result in the formation of a chimeric RNA molecule encoding a complete first reporter molecule; (iv) a nucleic acid sequence encoding a different, second reporter molecule and (v) an internal ribosome entry site (IRES), whereby the expression of the nucleic acid sequence encoding the second reporter molecule will be driven off the IRES; but wherein at least one structural element of the candidate PTMs, selected from (a) target-binding domain, (b) 3' splice region and/or 5' splice region, (c) optional spacer region that separates the splice region from the target-binding domain and (d) optional safety sequence, is replaced library-wide by random or a multiplicity of nucleotide sequences;
(II) selecting cells expressing the first and second reporter molecules, thus indicating the presence of a PTM capable of mediating a trans-splicing reaction in the selected cell and the specificity of the trans-splicing; and
(III) identifying the PTM expressed in the selected cells.

2. The method of Claim 1, wherein the reporter molecules are selected from bioluminescent, fluorescent, a receptor, an enzyme and a protein/peptide tag.

3. The method of Claim 1 or 2, wherein the reporter molecules are fluorescent and the cells expressing optimal PTMs are selected by fluorescent cell sorting.

4. The method of any preceding Claim, wherein expression of the target pre-mRNA is under the control of an inducible promoter.

5. An expression library of candidate PTMs which comprise (i) a target-binding domain; (ii) a 3'-splice region and/or a 5'-splice region, and (iii) at least one nucleic acid sequence encoding a portion of a reporter molecule, so that a successful trans-splicing reaction will result in the formation of a chimeric RNA molecule encoding a complete reporter molecule, (iv) a nucleic acid sequence encoding a different, second reporter molecule, and (v) an internal ribosome entry site (IRES), whereby the expression of the nucleic acid sequence encoding the second reporter molecule will be driven off the IRES but wherein at least one structural element of the candidate PTMs, selected from (a) target-binding domain, (b) 3' splice region and/or 5' splice region, (c) optional spacer region that separates the splice region from the target-binding domain and (d) optional safety sequence, is replaced library-wide by random or a multiplicity of nucleotide sequences.

6. The expression library of Claim 5, wherein the reporter molecules are selected from bioluminescent, fluorescent, a receptor, an enzyme and a protein/peptide tag.

7. A culture of mammalian cells which contain members of an expression library as defined in Claim 5 or 6.

## Patentansprüche

1. Verfahren zur Identifizierung optimaler prä-Trans-Spleißmoleküle (PTMs), die mit einer Target-prä-mRNA wechselwirkungsfähig sind und eine Trans-Spleißreaktion damit vermitteln können, die zur Erzeugung eines chimären RNA-Moleküls führt, wobei das Verfahren aufweist:
(I) Inkontaktbringen einer Expressionsbibliothek von Kandidaten-PTMs mit Zellen, die eine Target-prä-mRNA exprimieren, unter Bedingungen, bei denen eine derartige trans-Spleißreaktion in Anwesenheit eines oder mehrerer PTMs auftreten wird, wobei die Kandidaten-PTMs aufweisen: (i) eine target-bindende Domäne; (ii) eine 3'-Spleißregion und/oder eine 5'-Spleißregion; (iii) mindestens eine Nucleinsäuresequenz, die für einen Abschnitt eines ersten Reportermoleküls codiert, so daß eine erfolgreiche trans-Spleißreaktion zur Bildung eines chimären RNA-Moleküls führt, das für ein vollständiges erstes Reportermolekül codiert; (iv) eine Nucleinsäuresequenz, die für ein anderes, zweites Reportermolekül codiert, und (v) eine interne ribosomale Eintrittsstelle (IRES-Element), wodurch die Expression der für das zweite Reportermolekül codierenden Nucleinsäuresequenz von dem IRES-Element weg verlagert wird, wobei aber mindestens ein Strukturelement der Kandidaten-PTMs, ausgewählt unter (a) der target-bindenden Domäne, (b) der 3'-Spleißregion und/oder 5'-Spleißregion, (c) einer wahlfreien Spacer-Region, welche die Spleiß-Region von der target-bindenden Domäne trennt, und (d) einer wahlfreien Sicherheitssequenz, bibliotheksweit durch zufällige oder eine Vielfalt von Nucleinsäuresequenzen ausgetauscht wird;
(II) Selektion von Zellen, welche die ersten und zweiten Reportermoleküle exprimieren, und folglich die Anwesenheit eines PTM, das imstande ist, eine trans-Spleißreaktion in der selektierten Zelle zu vermitteln, und die Spezifität des trans-Spleißvorgangs anzeigen; und
(III) Identifizierung des in den selektierten Zellen exprimierten PTM.

2. Verfahren nach Anspruch 1, wobei die Reportermoleküle unter biolumineszierenden, fluoreszierenden Molekülen, einem Rezeptor, einem Enzym und einem Protein/Peptid-Anhang ausgewählt sind.

3. Verfahren nach Anspruch 1 oder 2, wobei die Reportermoleküle fluoreszierend sind und die optimale PTMs exprimierenden Zellen durch Sortieren fluoreszierender Zellen selektiert werden.

4. Verfahren nach einem der vorstehenden Ansprüche, wobei die Expression der Target-prä-mRNA unter der Kontrolle eines induzierbaren Promotors erfolgt.

5. Expressionsbibliothek von Kandidaten-PTMs, die aufweisen: (i) eine target-bindende Domäne; (ii) eine 3'-Spleißregion und/oder eine 5'-Spleißregion und (iii) mindestens eine Nucleinsäuresequenz, die für einen Abschnitt eines Reportermoleküls codiert, so daß eine erfolgreiche trans-Spleißreaktion zur Bildung eines chimären RNA-Moleküls führt, das für ein vollständiges Reportermolekül codiert; (iv) eine Nucleinsäuresequenz, die für ein anderes, zweites Reportermolekül codiert, und (v) eine interne ribosomale Eintrittsstelle (IRES-Element), wodurch die Expression der für das zweite Reportermolekül codierenden Nucleinsäuresequenz von dem IRES-Element weg verlagert wird, wobei aber mindestens ein Strukturelement der Kandidaten-PTMs, ausgewählt unter (a) der target-bindenden Domäne, (b) der 3'-Spleißregion und/oder 5'-Spleißregion, (c) einer wahlfreien Spacer-Region, welche die Spleiß-Region von der target-bindenden Domäne trennt, und (d) einer wahlfreien Sicherheitssequenz, bibliotheksweit durch zufällige oder eine Vielfalt von Nucleinsäuresequenzen ausgetauscht wird.

6. Expressionsbibliothek nach Anspruch 5, wobei die Reportermoleküle unter biolumineszierenden, fluoreszierenden Molekülen, einem Rezeptor, einem Enzym und einem Protein/Peptid-Anhang ausgewählt sind.

7. Kultur von Säugetierzellen, die Elemente einer Expressionsbibliothek enthalten, wie in Anspruch 5 oder 6 definiert.

## Revendications

1. Procédé d'identification de molécules de pré-trans-épissage (les PTM) optimales capables d'interagir avec un pré-ARNm cible et de médier une réaction de trans-épissage avec celui-ci, débouchant sur la production d'une molécule d'ARN chimère, ledit procédé consistant à:
(I) mettre en contact une banque d'expression de molécules PTM candidates avec des cellules exprimant un pré-ARNm cible dans des conditions dans lesquelles une telle réaction de trans-épissage va se produire en présence d'une ou plusieurs PTM, lesdites molécules PTM candidates comprenant (i) un domaine de liaison à la cible; (ii) une région d'épissure en 3' et/ou une région d'épissure en 5'; (iii) au moins une séquence d'acide nucléique codant pour une partie d'une première molécule rapporteur, de sorte qu'une réaction de trans-épissage réussie débouchera sur la formation d'une molécule d'ARN chimère codant pour une première molécule rapporteur complète; (iv) une séquence d'acide nucléique codant pour une seconde molécule rapporteur, différente, et (v) un site d'entrée interne des ribosomes (l'IRES), grâce auquel la commande de l'expression de la séquence d'acide nucléique codant pour la seconde molécule rapporteur sera repoussée à l'IRES; mais où au moins un élément structural des molécules PTM candidates, choisi parmi (a) un domaine de liaison à la cible, (b) une région d'épissure en 3' et/ou une région d'épissure en 5', (c) une région d'espacement facultative qui sépare la région d'épissure et le domaine de liaison à la cible et (d) une séquence de sécurité facultative, est remplacé sur toute la banque par des séquences nucléotidiques aléatoires ou par une multiplicité de séquences nucléotidiques particulières;
(II) sélectionner des cellules qui expriment les première et seconde molécules rapporteurs, indiquant ainsi la présence d'une molécule PTM capable de médier une réaction de trans-épissage dans la cellule sélectionnée et la spécificité du trans-épissage; et
(III) identifier la molécule PTM exprimée dans les cellules sélectionnées.

2. Procédé selon la revendication 1, dans lequel les molécules rapporteurs sont choisies parmi une molécule bioluminescente, une molécule fluorescente, un récepteur, une enzyme et un marqueur protéique/peptidique.

3. Procédé selon la revendication 1 ou 2, dans lequel les molécules rapporteurs sont fluorescentes et les cellules exprimant les molécules PTM optimales sont sélectionnées par tri cellulaire par fluorescence (FCS).

4. Procédé selon l'une quelconque des revendications précédentes, dans lequel l'expression du pré-ARNm cible est sous le contrôle d'un promoteur inductible.

5. Banque d'expression de molécules PTM candidates qui comprennent (i) un domaine de liaison à la cible; (ii) une région d'épissure en 3' et/ou une région d'épissure en 5'; et (iii) au moins une séquence d'acide nucléique codant pour une partie d'une molécule rapporteur, de sorte qu'une réaction de trans-épissage réussie débouchera sur la formation d'une molécule d'ARN chimère codant pour une molécule rapporteur complète; (iv) une séquence d'acide nucléique codant pour une seconde molécule rapporteur, différente, et (v) un site d'entrée interne des ribosomes (l'IRES), grâce auquel la commande de l'expression de la séquence d'acide nucléique codant pour la seconde molécule rapporteur sera repoussée à l'IRES, mais où au moins un élément structural des molécules PTM candidates, choisi parmi (a) un domaine de liaison à la cible, (b) une région d'épissure en 3' et/ou une région d'épissure en 5', (c) une région d'espacement facultative qui sépare la région d'épissure et le domaine de liaison à la cible et (d) une séquence de sécurité facultative, est remplacé sur toute la banque par des séquences nucléotidiques aléatoires ou par une multiplicité de séquences nucléotidiques particulières.

6. Banque d'expression selon la revendication 5, dans laquelle les molécules rapporteurs sont choisies parmi une molécule bioluminescente, une molécule fluorescente, un récepteur, une enzyme et un marqueur protéique/peptidique.

7. Culture de cellules de mammifère qui contiennent des membres d'une banque d'expression telle que définie dans la revendication 5 ou 6.


### SEQUENCE LISTING

<110> MITCHELL, Lloyd G.
   PUTTARAJU, Madaiah
   GARCIA-BLANCO, Mariano
   OTTO, Edward
   YANG, Yanping
<120> SCREENING METHODS FOR IDENTIFICATION OF EFFICIENT
   PRE-TRANS-SPLICING MOLECULES
<130> 027705.00059 PCT EPC
<140> PCT/US2003/034102
   <141> 2003-10-23
<150> US 60/420,498
   <151> 2002-10-23
<160> 7
<170> PatentIn version 3.3
<210> 1
   <211> 28
   <212> DNA
   <213> Artificial
<220>
   <223> PTM Cassette(1)
<400> 1
   tcagatccgc tagcgtttaa acccgcgg 28
<210> 2
   <211> 41
   <212> DNA
   <213> Artificial
<220>
   <223> PTM Cassette
<400> 2
   tactaactca attttttttt tttttttttt aattaacagg t 41
<210> 3
   <211> 8
   <212> DNA
   <213> Artificial
<220>
   <223> PTM Cassette
<400> 3
   ggcgcgcc 8
<210> 4
   <211> 24
   <212> DNA
   <213> Artificial
<220>
   <223> PTM Cassette
<400> 4
   acgatctcat attctatcgt cgaa 24
<210> 5
   <211> 18
   <212> DNA
   <213> Artificial
<220>
   <223> 3' ss
<400> 5
   atgctaacta ggcgaggt 18
<210> 6
   <211> 20
   <212> DNA
   <213> Artificial
<220>
   <223> PTM cassette
<400> 6
   gctagcgttt aaacccgcgg 20
<210> 7
   <211> 45
   <212> DNA
   <213> Artificial
<220>
   <223> PTM cassette
<400> 7
   tactaactca attttttttt tttttttttt aattaacagg gtttt 45